# EUROPEAN PATENT APPLICATION

(11) **EP 4 636 080 A1**
(43) Date of publication of application: **22.10.2025**
(21) Application number: 23902786.5
(22) Date of filing: 14.12.2023
(51) Int. Cl.: C12N 5/10, C12N 5/0735, C12N 5/074

(54) **UNIVERSAL CELL AND PREPARATION METHOD THEREFOR**

(30) Priority: 16.12.2022 CN 202211627210
(71) Applicant: Xellsmart Bio-Pharmaceutical (Suzhou) Co., Ltd., Suzhou, Jiangsu 210008 (CN)
(72) Inventor: LI, Xiang, Shanghai 201100 (CN); ZHU, Minzhe, Shanghai 201100 (CN)
(74) Representative: Gille Hrabal Partnerschaftsgesellschaft mbB Patentanwälte
(86) International application number: PCT/CN2023/138705
(87) International publication number: WO 2024/125592

(57) **Abstract**

Disclosed are a universal cell and a preparation method therefor. After major histocompatible complex MHC-I and II genes are inactivated in cells, at least one protein of SLC35A1, GNE, CMAS, ST3Gal5, ST8SIA1, MUC1, APMAP, SMAGP, and CD43 is overexpressed, so that the obtained human pluripotent stem cells or human cell lines can further escape the killing of NK cells and macrophages on the basis of escaping the attack of T cells. Meanwhile, the low-immunogenicity pluripotent stem cells retain the stemness and differentiation capacity thereof.

## Description

### CROSS-REFERENCE TO RELATED APPLICATION

The present application claims the priority to and the benefits of the Chinese Patent Application No. 202211627210.4 filed on December 16, 2022, which is incorporated herein by reference in its entirety.

### TECHNICAL FIELD

The present disclosure pertains to the cross-disciplinary field of genetic engineering and stem cell technology, and particularly relates to a universal cell and a preparation method therefor.

### BACKGROUND

Through *in vitro* culture of cells or induced differentiation of stem cells, healthy functional cells can be regenerated in large quantities *in vitro,* thereby treating diseases through allogeneic functional cell transplantation. However, immune incompatibility and immunological rejection face31d by transplanted cells remain critical barriers to the clinical application of cell-based therapy. Stem cells are a class of "seed" cells capable of self-renewal and differentiation into specific functional somatic cells. Based on the differences in the degrees of stem cell characteristics, stem cells are primarily classified into: totipotent stem cells, pluripotent stem cells (PSCs), andadult stem cells. Human embryonic stem cells (hESCs) and induced pluripotent stem cells (iPSCs) have the potential for unlimited proliferation, self-renewal, and differentiation into diverse cell types, thereby exhibiting good application prospects in the treatment of related diseases such as cancer, neurological disorders, cardiovascular diseases, and the like.

Autologous cell transplantation therapy can circumvent immunological rejection issues. However, producing autologous cells from patients is cost-prohibitive and involves lengthy preparation processes (Khera et al., 2013), and individual-derived cell products may exhibit uncertain quality and effectiveness. There are data indicating that cells from patients exhibit differences compared to those from normal individuals, which may affect the therapeutic efficacy of autologous cell-based therapy. Additionally, studies have shown that immunosuppressive drugs, HLA matching, and gene editing can reduce immunogenicity or mitigate the host immune system's rejection of allogeneic transplanted cells. Immunosuppressive drugs are associated with significant side effects, and may cause myelosuppression, hepatotoxicity, alopecia, and gastrointestinal adverse reactions. Currently, the United States, Japan, and China are establishing HLA-matched iPSC banks. However, both bank establishment and maintenance require substantial costs, as the genes for HLA antigens are the most polymorphic genes observed in the human genome. Existing iPSC banks in various countries and regions are unable to provide matches for the majority of their respective populations, and can only cover specific population groups (Solomon et al., 2015; Turner et al., 2013). Allogeneic cell therapy for large patient populations may offer significant advantages over HLA-matched cell banks in terms of economy and construction and operational costs. However, allogeneic cell therapy is prone to severe immunological rejection. Therefore, the construction ofimmune-compatible universal allogeneic PSCs has become an urgent imperative.

In humans, the major histocompatibility complex (MHC), also known as the human leukocyte antigen (HLA), is the primary cause of immune incompatibility. The HLA complex consists of a series of genes that can be classified into class I, class II, and class III. MHC-I genes are expressed in almost all tissue cell types. Transplanted cells expressing "non-self" MHC class I molecules can stimulate the activation of CD8+ T cells and thus be eliminated. CD4+ helper T cells recognize the MHC-II genes of "non-self" cells, thereby triggering immunological rejection, while class III molecules do not participate in immune activities. By utilizing gene editing methods to modify immunogenic elements, low-immunogenicity cells can be produced, enabling the large-scale production of "off-the-shelf" cell therapy products with immune privilege.

In recent years, pioneering reports have demonstrated that by knocking out genes such as B2M and CIITA, expression of MHC-I and MHC-II on cell surfaces or genes themselves can be eliminated, so that the cells can have immune tolerance or escape T/B cell-specific immune responses, and thus immune-compatible universal PSCs can be generated, thereby laying an important foundation for broader applications of universal PSC-derived cells, tissues, and organs. However, HLA molecules serve as the primary inhibitory ligands for natural killer (NK) cells, and MHC class I-negative cells are susceptible to NK cell-mediated lysis. Both *in vitro* and *in vivo* data have shown that host NK cells can eliminate transplanted B2M^{-/-} donor cells (Flahou et al., 2021). Thus, previous methods require refinement to produce optimized universal donor cells that can evade immune responses.

### SUMMARY

Aiming at the shortcomings of the prior art, the present disclosure provides a novel method for engineering cells to achieve low immunogenicity. For the first time, large-scale screening of genes associated with sialic acid-related protein expression is conducted. Through multiple functional assays, 9 representative novel genes, SLC35A1, GNE, CMAS, ST3Gal5, ST8SIA1, MUC1, APMAP, SMAGP, and CD43, are finally identified. These genes can significantly reduce or escape the recognition and attack by the immune system, especially the attack by natural killer cells, macrophages, etc. The present disclosure proposes, for the first time, a feasible cross-disciplinary application strategy for the use of sialic acid-related protein genes in cell engineering to achieve cells with immune privilege, which has demonstrated unexpected excellent technical effects.

According to the present disclosure, B2M/CIITA double allele knockout (DKO) positive clone cells are successfully constructed by knocking out β-2-microglobulin (B2M) in the endoplasmic reticulum of human pluripotent stem cells and knocking out CIITA (a positive regulatory factor for MHC-II gene transcription); and then the novel gene identified in the present disclosure (SLC35A1, GNE, CMAS, ST3Gal5, ST8SIA1, MUC1, APMAP, SMAGP, or CD43) is overexpressed in the DKO cells by using a lentiviral vector. The resulting human pluripotent stem cells can not only escape the attack by T cells but further escape killing by NK cells and macrophages. Meanwhile, these low-immunogenicity pluripotent stem cells retain their critical biological functions of pluripotent stem cells, such as stemness, differentiation ability, etc.

In order to achieve the above objectives, the present disclosure adopts the following technical solutions:
In a first aspect, the present disclosure provides a universal cell, relative to a wild-type cell, which comprises:
1) reduced expression or absent expression of MHC-I and/or MHC-II human leukocyte antigens, and
2) an expression sequence of at least one of SLC35A1, GNE, CMAS, ST3Gal5, ST8SIA1, MUC1, APMAP, SMAGP, and CD43 genes, with the expression level of the gene being increased, wherein the cell is capable of escaping the attack by T cells and the killing by NK cells and macrophages.

In certain aspects, the cell comprises reduced expression or absent expression of MHC-I and MHC-II human leukocyte antigens.

In certain aspects, the cell further comprises a modification to increase the expression of one or more polypeptides selected from: DUX4, CD27, CD35, CD200, HLA-C, PD-L1, CD47, CD24, CD26, CCL21, Mfge8, and SerpinB9.

In certain aspects, a gene editing tool (such as TALEN and/or CRISPR systems) is used in the cell to target one or more genes encoding one or more transcription factors of MHC-I, and one or more genes encoding one or more transcription factors of MHC-II, thereby achieving the reduced expression or absent expression of MHC-I and MHC-II genes.

In certain embodiments, to achieve the reduced expression or absent expression of MHC-I and MHC-II genes, the transcription factor of MHC-I may preferably be selected from one or more of B2M, TAP1, TAP2, TAP-associated glycoprotein (Tapasin), or NLRC5; the transcription factor of MHC-II may preferably be selected from one or more of CIITA, RFXANK, RFX5, and RFXAP;
the transcription factors are more preferably B2M and CIITA.

In certain embodiments, the cell further comprises a genetic modification targeting the CIITA gene through a rare-cutting endonuclease that selectively inactivates the CIITA gene.

In certain embodiments, the cell further comprises a genetic modification targeting the B2M gene through a rare-cutting endonuclease that selectively inactivates the B2M gene.

In certain embodiments, the rare-cutting endonuclease is selected from a CAS protein, a TALE-nuclease, a zinc finger nuclease, a meganuclease, and a homing nuclease.

In certain embodiments, the genetic modification targeting the CIITA gene or the B2M gene through the rare-cutting endonuclease comprises a CAS protein or a polynucleotide encoding a CAS protein, and at least one guide ribonucleic acid sequence for specifically targeting the CIITA gene or the B2M gene.

In a specific embodiment, a CRISPR/CAS9 system is used to directly knock out both ends of exons of B2M and CIITA separately, wherein target sequences of gRNA for the B2M gene are SEQ ID NO: 22 and SEQ ID NO: 23, and target sequences of gRNA for the CIITA gene are SEQ ID NO: 24 and SEQ ID NO: 25.

In certain aspects, a gene expression-modifying molecule for one or more genes encoding one or more transcription factors of MHC-I, or for one or more genes encoding one or more transcription factors of MHC-II is introduced into the cell, thereby achieving the reduced expression or absent expression of MHC-I and/or MHC-II genes, wherein the gene expression-modifying molecule comprises one selected from an siRNA, an shRNA, a microRNA, an antisense RNA, and another RNA-mediated inhibitory molecule.

In certain aspects, the amino acid sequence of the SLC35A1, GNE, CMAS, ST3Gal5, ST8SIA1, MUC1, APMAP, SMAGP, or CD43 has 70% or more homology, for example, 80% or more homology, for another example, 90% or more, 95% or more, 98% or more, or 99% or more homology, to the sequence set forth in SEQ ID NO: 1, 3, 4, 8, 10, 12, 16, 17, or 19, respectively;
further preferably, the amino acid sequence of the SLC35A1, GNE, CMAS, ST3Gal5, ST8SIA1, MUC1, APMAP, SMAGP, or CD43 is set forth in SEQ ID NO: 1, 3, 4, 8, 10, 12, 16, 17, or 19, respectively;
still further preferably, the universal cell comprises an expression sequence of at least one of MUC1, APMAP, and CD43 genes;
yet still further preferably, the universal cell comprises an expression sequence of CD43.

In certain aspects, the cell is an embryonic stem cell.

In certain aspects, the cell is a pluripotent stem cell.

In certain embodiments, the cell is a low-immunogenicity stem cell.

In certain embodiments, the cell is a human stem cell or a human somatic cell.

In a second aspect, the present disclosure provides a preparation method for the universal cell according to the first aspect, which comprises the following steps:
1) knocking out one or more genes of one or more transcription factors of MHC-I in a cell; and/or
2) knocking out one or more genes of one or more transcription factors of MHC-II in the cell; and
3) introducing into the cell a nucleic acid sequence encoding at least one of SLC35A1, GNE, CMAS, ST3Gal5, ST8SIA1, MUC1, APMAP, SMAGP, and CD43 proteins.

In certain aspects, the transcription factor of MHC-I may be preferably selected from one or more of B2M, TAP1, TAP2, TAP-associated glycoprotein (Tapasin), or NLRC5; the transcription factor of MHC-II may preferably be selected from one or more of CIITA, RFXANK, RFX5, and RFXAP.

In certain embodiments, the transcription factors are selected from B2M and CIITA.

In certain aspects, the knocking out in steps 1) and 2) is a genetic modification targeting the CIITA gene or the B2M gene through a rare-cutting endonuclease that selectively inactivates the CIITA gene or the B2M gene.

Preferably, the rare-cutting endonuclease is selected from a CAS protein, a TALE-nuclease, a zinc finger nuclease, a meganuclease, and a homing nuclease.

Further preferably, the genetic modification targeting the CIITA gene or the B2M gene through the rare-cutting endonuclease comprises a CAS protein or a polynucleotide encoding a CAS protein, and at least one guide ribonucleic acid sequence for specifically targeting the CIITA gene or the B2M gene.

In certain embodiments, in steps 1) and 2), a CRISPR system is used to directly knock out both ends of exons of B2M and CIITA separately, wherein target sequences of gRNA for the B2M gene are SEQ ID NO: 22 and SEQ ID NO: 23, and target sequences of gRNA for the CIITA gene are SEQ ID NO: 24 and SEQ ID NO: 25.

In certain aspects, the knocking out in step 1) or 2) is performed by introducing a gene expression-modifying molecule for one or more genes encoding one or more transcription factors of MHC-I, or for one or more genes encoding one or more transcription factors of MHC-II, thereby achieving the reduced expression or absent expression of MHC-I and/or MHC-II genes, wherein the gene expression-modifying molecule comprises one selected from an siRNA, an shRNA, a microRNA, an antisense RNA, and another RNA-mediated inhibitory molecule.

In certain aspects, in step 3), a gene knock-in approach is used to introduce into the cell the nucleic acid sequence encoding at least one of SLC35A1, GNE, CMAS, ST3Gal5, ST8SIA1, MUC1, APMAP, SMAGP, and CD43 proteins.

In certain aspects, in step 3), an expression vector is used to introduce into the cell the nucleic acid sequence encoding at least one of SLC35A1, GNE, CMAS, ST3Gal5, ST8SIA1, MUC1, APMAP, SMAGP, and CD43 proteins.

Preferably, the expression vector used in step 3) is a viral vector.

In certain embodiments, the viral vector used in step 3) is a lentivirus.

In certain aspects, in step 3), the nucleic acid sequence encoding at least one of SLC35A1, GNE, CMAS, ST3Gal5, ST8SIA1, MUC1, APMAP, SMAGP, and CD43 proteins is introduced into a selected site of the cell; preferably, the selected site of the cell is a safe harbor gene site.

In certain aspects, the amino acid sequence of the SLC35A1, GNE, CMAS, ST3Gal5, ST8SIA1, MUC1, APMAP, SMAGP, or CD43 has 70% or more homology, for example, 80% or more homology, for another example, 90% or more, 95% or more, 98% or more, or 99% or more homology, to the sequence set forth in SEQ ID NO: 1, 3, 4, 8, 10, 12, 16, 17, or 19, respectively;
further preferably, the amino acid sequence of the SLC35A1, GNE, CMAS, ST3Gal5, ST8SIA1, MUC1, APMAP, SMAGP, or CD43 is set forth in SEQ ID NO: 1, 3, 4, 8, 10, 12, 16, 17, or 19, respectively;
still further preferably, in step 3), the expression vector is used to introduce into the cell the nucleic acid sequence encoding at least one of MUC1, APMAP, and CD43 proteins;
yet still further preferably, in step 3), the expression vector is used to introduce into the cell the nucleic acid sequence encoding CD43.

In certain aspects, the universal cell further comprises a second expression vector comprising a polynucleotide sequence encoding one or more selected from DUX4, CD27, CD35, CD200, HLA-C, PD-L1, CD47, CD24, CD26, CCL21, Mfge8, and SerpinB9.

In certain embodiments, the second expression vector is an inducible expression vector; preferably, the second expression vector is a viral vector.

In a third aspect, the present disclosure provides a method for preparing a differentiated universal cell, which comprises culturing the universal cell prepared according to the method of the second aspect under differentiation conditions, thereby preparing a differentiated low-immunogenicity cell. In certain aspects, the differentiation conditions are suitable for differentiating the cell into a cell type selected from a cardiomyocyte, a nerve cell, a glial cell, an endothelial cell, a T cell, an NK cell, an NKT cell, a macrophage, a hematopoietic progenitor cell, a mesenchymal cell, an islet cell, a chondrocyte, a retinal pigment epithelial cell, a renal cell, a hepatocyte, a thyroid cell, a skin cell, a blood cell, and an epithelial cell.

In a fourth aspect, the present disclosure provides a method of treatment, which comprises administering to a patient suitable for cell therapy a population of differentiated low-immunogenicity cells prepared according to the method of the third aspect.

In a fifth aspect, the present disclosure provides a composition, which comprises the universal cell according to the first aspect.

In certain aspects, the composition comprises the universal cell according to the first aspect and one or more therapeutic agents, wherein the therapeutic agent comprises a peptide, a cytokine, a small-molecule compound, a macromolecule, an ADC, an antibody, a nanoparticle, a biosimilar, an mRNA, a traditional Chinese medicine, a protein, a vaccine, a checkpoint inhibitor, a mitogen, a growth factor, a small RNA, a double-stranded RNA (dsRNA), a mononuclear blood cell, a feeder cell, a feeder cell component or a replacement factor thereof, a vector comprising one or more polynucleic acids of interest, etc.

In a sixth aspect, the present disclosure provides a cell having increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In a seventh aspect, the present disclosure provides a cell having absent expression of CIITA, increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In an eighth aspect, the present disclosure provides a cell having absent expression of B2M, increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In a ninth aspect, the present disclosure provides a cell having absent expression of CIITA and B2M, increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In a tenth aspect, the present disclosure provides a cell having increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In an eleventh aspect, the present disclosure provides a cell having absent expression of CIITA, increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In a twelfth aspect, the present disclosure provides a cell having absent expression of B2M, increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

In a thirteenth aspect, the present disclosure provides a cell having absent expression of CIITA and B2M, increased expression of at least one of SLC35A1, GNE, CMAS, ST3Gal5, ST8SIA1, MUC1, APMAP, SMAGP, or CD43 protein, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

The cell according to the sixth to thirteenth aspects described above is selected from a stem cell, a differentiated cell, a pluripotent stem cell, an induced pluripotent stem cell, an adult stem cell, a progenitor cell, a somatic cell, a primary T cell, and a chimeric antigen receptor T cell.

In a fourteenth aspect, the present disclosure provides use of the universal cell according to the first aspect, the composition according to the fifth aspect, or the cells according to the sixth to thirteenth aspects in the preparation of a product for cell therapy.

In a fifteenth aspect, the present disclosure provides use of the universal cell according to the first aspect, the composition according to the fifth aspect, or the cells according to the sixth to thirteenth aspects in the preparation of a product for organ transplantation.

In a sixteenth aspect, the present disclosure provides use of the universal cell according to the first aspect, the composition according to the fifth aspect, or the cells according to the sixth to thirteenth aspects in the construction of a cell bank of universal PSCs.

In a seventeenth aspect, the present disclosure provides use of the universal cell according to the first aspect, the composition according to the fifth aspect, or the cells according to the sixth to thirteenth aspects as a gene drug carrier.

In an eighteenth aspect, the present disclosure provides a kit for differentiating the universal cell according to the first aspect into a low-immunogenicity cardiomyocyte, which comprises a first medium and a second medium, wherein
the first medium is a basal medium comprising a GSK-3 inhibitor;
the second medium is a basal medium comprising a Wnt inhibitor.

In certain embodiments, the kit comprises a basal medium; and/or
the GSK-3 inhibitor is selected from CHIR99021; and/or
the Wnt inhibitor is selected from any one, two, or more of IWR-1, IWP-2, IWP-4, and C59; and/or
the concentration of the GSK-3 inhibitor is 0.5 µM-10 µM, preferably 6 µM; and/or
the concentration of the Wnt inhibitor is 0.5 µM-20 µM, preferably 2 µM.

In certain embodiments, the basal medium is selected from a PRMI1640 medium, an mTESR1 medium, etc.; further preferably, the basal medium is a PRMI1640 medium supplemented with insulin-containing B27.

In a nineteenth aspect, the present disclosure provides a method for differentiating the universal cell according to the first aspect into a low-immunogenicity cardiomyocyte, which comprises the following steps:
(a) culturing the stem cells in a differentiation medium supplemented with a GSK-3 inhibitor to induce differentiation of the stem cells into cardiomyocytes;
(b) continuing the induced differentiation in a differentiation medium;
(c) continuing the induced differentiation in a differentiation medium supplemented with a Wnt inhibitor; and
(d) continuing the induced differentiation in a differentiation medium.

In certain embodiments, the method comprises the following steps:
(a) on days 0-2, culturing the stem cells in a differentiation medium supplemented with a GSK-3 inhibitor to induce differentiation of the stem cells into cardiomyocytes;
(b) after 1 day of induction, continuing the induced differentiation in a basal medium;
(c) on days 3-5, continuing the induced differentiation in a differentiation medium supplemented with a Wnt inhibitor; and
(d) on days 5-15, continuing the induced differentiation in a basal medium.

In certain embodiments, in steps (a), (b), (c), and (d), the differentiation medium comprises a PRMI1640 basal medium and an insulin-free B27 supplement.

In certain embodiments, the GSK-3 inhibitor is selected from CHIR99021; and/or
the Wnt inhibitor is selected from any one, two, or more of IWR-1, IWP-2, IWP-4, and C59.

In certain embodiments, the concentration of the GSK-3 inhibitor is 0.5 µM-10 µM, preferably 6 µM; and/or
the concentration of the Wnt inhibitor is 0.5 µM-20 µM, preferably 2 µM.

In certain embodiments, in step (a), when the universal cells grow to a confluence of 85%-98% (e.g., 95%), the medium is replaced with the differentiation medium to induce differentiation.

In certain embodiments, in step (d), spontaneously beating cells may be observed on day 9.

In certain embodiments, the stem cells include pluripotent stem cells, multipotent stem cells, and unipotent stem cells.

In certain embodiments, the stem cells are human pluripotent stem cells, such as human embryonic stem cells or human induced pluripotent stem cells.

In certain embodiments, the product is selected from a reagent, a composition, a medium, etc.

In a twentieth aspect, the present disclosure provides a cell therapeutic agent for preventing or treating a heart disease, which comprises the differentiated mature cardiomyocytes prepared by the method described above.

In certain embodiments, the heart disease may be at least one selected from the group consisting of, but not limited to: myocardial infarction, angina pectoris, ischemic cardiomyopathy, primary cardiomyopathy, secondary cardiomyopathy, and heart failure.

### Beneficial Effects of Present Disclosure:

According to the present disclosure, after major histocompatibility complex (MHC) class I and II genes are inactivated in stem cells, at least one of SLC35A1, GNE, CMAS, ST3Gal5, ST8SIA1, MUC1, APMAP, SMAGP, and CD43 proteins is overexpressed, so that resulting human pluripotent stem cells can not only escape the attack by T cells but further escape killing by NK cells and macrophages. Among them, MUC1 (0.736), APMAP (0.744), and CD43 (0.520) exhibit superior relative killing ratios, all less than 0.75; DKO+CD43 cells emerge as theoptimal cell line for escaping NK cell killing, not only showing consistently lower rates compared to DKO under multiple ratios and across repeated killing experiments, but also demonstrating a superior average killing rate compared to the positive control WT (0.581) and the prior art DKO+CD47 (0.579); iPSC-DKO+CD43 cells can significantly escape killing by MAC cells, with superior escape efficacy compared to iPSC-WT and iPSC-DKO cells. An unexpected transformation effect is achieved. Meanwhile, these low-immunogenicity pluripotent stem cells retain their stemness and differentiation ability.

### BRIEF DESCRIPTION OF THE DRAWINGS

FIG. 1 shows a B2M gene knockout strategy in the human embryonic stem cell line H1 and the human induced pluripotent stem cell line iPSC (A), and the corresponding results (B and C).
FIG. 2 shows a CIITA gene knockout strategy in the human embryonic stem cell line H1 and the human induced pluripotent stem cell line iPSC (A), and the corresponding results (B and C).
FIG. 3 shows the expression of B2M and CIITA at the RNA level in H1 DKO cells, as detected by RT-qPCR in Example 1.
FIG. 4 shows the result of the B2M protein level in H1 DKO cells, as detected by Western-blot in Example 1.
FIG. 5 shows the expression of HLA-I/II in various cells including wild-type H1 WT and iPSC WT cells, and DKO cells thereof after INF-gamma stimulation, as detected by using a flow cytometer in Example 1, where
   the T cells serve as a positive control for detecting HLA class I/II molecules.
FIG. 6 shows the karyotype of B2M/CIITA double allele knockout (DKO) positive clones of H1 cells obtained in Example 1.
FIG. 7 shows the expression of pluripotency genes POU5F1/NANOG/SOX2 at the RNA and protein levels in H1 WT and DKO cells, as detected by immunofluorescence (A) and RT-qPCR (B) in Example 2, where
   MSCs serve as a negative control.

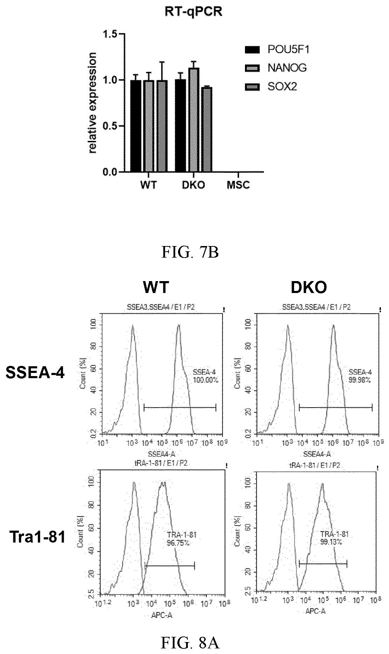

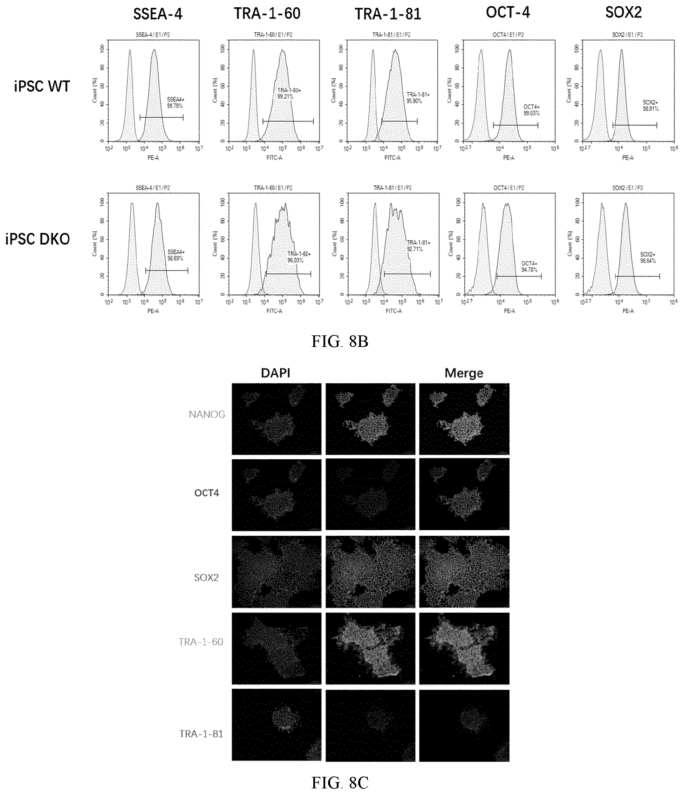

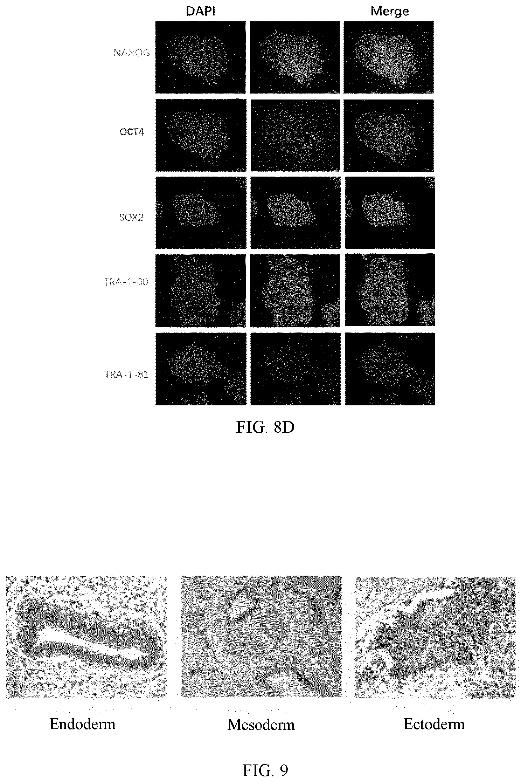

FIG. 8 shows the analysis of WT and DKO cells of H1 cells and iPSCs via immunofluorescence and flow cytometry in Example 2, where FIG. 8A shows the flow cytometry results of the expression of pluripotency genes SSEA-4 and Tra1-81 on the surface of H1 WT and H1 DKO cells, FIG. 8B shows the flow cytometry results of the expression of pluripotency genes SSEA-4, TRA-1-60, TRA-1-81, OCT-4, and SOX2 on the surface of iPSC-WT and iPSC-DKO cells, FIG. 8C shows the immunofluorescence results of the protein levels of pluripotency genes TRA-1-60, TRA-1-81, OCT-4, SOX2, and NANOG in iPSC-WT cells, and FIG. 8D shows the immunofluorescence results of the protein levels of pluripotency genes TRA-1-60, TRA-1-81, OCT-4, SOX2, and NANOG in iPSC-DKO cells.
FIG. 9 shows the formation of teratomas having three germ layers (endoderm, mesoderm, and ectoderm) by H1 DKO cells, as demonstrated by immunohistochemistry.

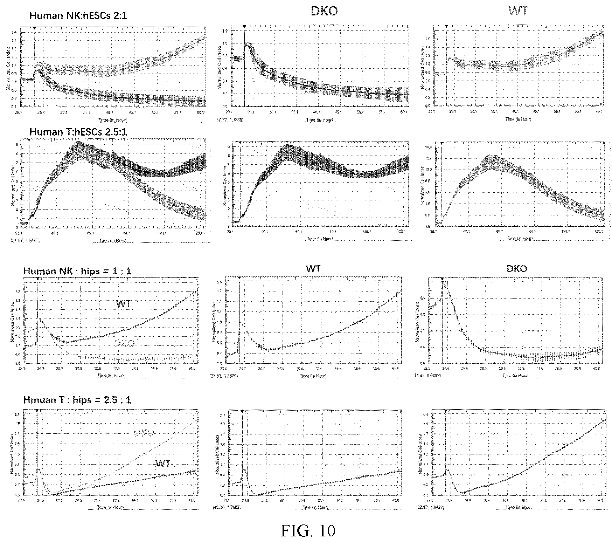

FIG. 10 shows the results of immune function validation for WT and DKO cells, as detected by RTCA in Example 2, where the first row of three panels shows the RTCA results of the killing rates of NK cells against H1 WT and H1 DKO cells, i.e., the detection results of the immune escape function of H1 WT and H1 DKO cells against NK cells; the second row of three panels shows the RTCA results of the killing rates of T cells against H1 WT and H1 DKO cells, i.e., the detection results of the immune escape function of WT and DKO cells against T cells; the third row of three panels shows the RTCA results of the killing rates of NK cells against iPSC-WT and iPSC-DKO cells, i.e., the detection results of the immune escape function of iPSC-WT and iPSC-DKO cells against NK cells; and the fourth row of three panels shows the RTCA results of the killing rates of T cells against iPSC-WT and iPSC-DKO cells, i.e., the detection results of the immune escape function of iPSC-WT and iPSC-DKO cells against T cells.
FIG. 11 shows a schematic structural diagram of a pGC-EF1a plasmid.
FIG. 12 shows the expression of CD47 in the H1 DKO+CD47 cell line constructed in Example 3, as detected by using a flow cytometer.
FIG. 13 shows the results of the killing of the CD47-overexpressing H1 DKO+CD47 cell strain, H1 WT cells, and H1 DKO cells by NK cells, as detected by RTCA in Example 3.
FIG. 14 shows the results of the killing of the candidate protein-overexpressing cell strains, H1 WT cells (positive control), and H1 DKO cells (negative control) by NK cells, as detected by RTCA in example 4.
FIG. 15 shows the mRNA overexpression level relative to DKO in the constructed H1 DKO+CD43 cell strain, as detected by qPCR in Example 5.
FIG. 16 shows the expression of pluripotency genes in the constructed H1 DKO+CD43 cell strain, as detected by immunofluorescence (A) and flow cytometry (B) in Example 5.
FIG. 17 shows the results of the expression of ectoderm, mesoderm, and endoderm marker proteins at the protein level in H1 DKO+CD43 cells, as detected by immunofluorescence in Example 5.
FIG. 18 shows the formation of teratomas having three germ layers (endoderm, mesoderm, and ectoderm) by H1 DKO+CD43 cells,
   as demonstrated by immunohistochemistry in Example 5.
FIG. 19 shows the results of the killing of CD43 protein-overexpressing H1 DKO+CD43 cell strain, H1 WT cells, H1 DKO cells, H1 DKO+CD47 cells, and H1 DKO+CD24 cells by NK cells (A), along with the summary of multiple repeated experiments normalized to DKO killing results in each experiment (B); and the killing results of iPSC WT cells, iPSC DKO cells, and iPSC DKO+CD43 cell strain (C), along with the summary of multiple repeated experiments normalized to DKO killing results in each experiment (D), as detected by RTCA in Examples 6 and 8.
FIG. 20 shows the results of the killing of CD43 protein-overexpressing H1 DKO+CD43 cell strain, H1 WT cells, H1 DKO cells, H1 DKO+CD47 cells, and H1 DKO+CD24 cells by mixed lymphocytes (PBNK cells) (A), along with the summary of multiple repeated experiments normalized to DKO killing results in each experiment (B); and the killing results of iPSC WT cells, iPSC DKO cells, and iPSC DKO+CD43 cell strain (C), along with the summary of multiple repeated experiments normalized to DKO killing results in each experiment (D), as detected by RTCA in Examples 7 and 8.
FIG. 21 shows the detection results of the escape of H1 WT cells, H1 DKO cells, H1 DKO+CD43 cells, H1 DKO+CD47 cells, and H1 DKO+CD24 cells from PBNK cell killing after 24-hour co-culture with PBNK cells in Example 7.
FIG. 22 shows the results of the number of spots formed by IFN-γ secreted by PBNK cells stimulated by 24-hour co-culture with H1 WT cells, H1 DKO cells, H1 DKO+CD43 cells, H1 DKO+CD47 cells, and H1 DKO+CD24 cells, as detected by Elispot in Example 7.
FIG. 23 shows the results of the CD107a degranulation levels in CD8+ T cells and NK cells within mixed lymphocytes (PBNK cells) after 4-hour co-culture with H1 WT cells, H1 DKO cells, H1 DKO+CD43 cells, H1 DKO+CD47 cells, and H1 DKO+CD24 cells (A and B), as detected by FACS in Example 7.
FIG. 24 shows the results of the escape of iPSC WT cells, iPSC DKO cells, and iPSC DKO+CD43 cells from macrophage killing after 24-hour co-culture with PBNK cells (A), along with the summary of multiple repeated experiments normalized to DKO killing results in each experiment (B), as detected by RTCA in Example 8.
FIG. 25 shows the results of the escape of DKO+CD43 differentiated cells from NK cell killing (A), along with the summary of multiple repeated experiments normalized to DKO killing results in each experiment (B), as detected by RTCA in Example 9.
FIG. 26 shows the results of the escape of DKO+CD43 differentiated cells from PBNK cell killing (A), along with the summary of multiple repeated experiments normalized to DKO killing results in each experiment (B), as detected by RTCA in Example 9.
FIG. 27 shows the results of the escape of DKO+CD43 differentiated cells from macrophage killing (A), along with the summary of multiple repeated experiments normalized to DKO killing results in each experiment (B), as detected by RTCA in Example 9.
FIG. 28 shows the immunofluorescence detection of pluripotency gene proteins in NHP-iPSCs (A); and the formation of teratomas having three germ layers (endoderm, mesoderm, and ectoderm), as demonstrated by immunohistochemistry.
FIG. 29 shows the expression of HLA-I/II in various cells including NHP iPSC-WT and NHP iPSC-DKO cells after INF-gamma stimulation, as detected by using a flow cytometer in Example 10. **Note: The labels NHP WT, NHP DKO1, NHP DKO2, and NHP DKO+CD43 in** **FIGs. 30-33** **below correspond to NHP iPSC-WT, NHP iPSC-DKO1, NHP iPSC-DKO2, and NHP iPSC-DKO+CD43, respectively.**
FIG. 30 shows the results of the killing of NHP iPSC-WT and NHP iPSC-DKO cells by monkey T cells, as detected by RTCA in Example 10.
FIG. 31 shows the detection of overexpression in NHP iPSC-DKO+CD43 in Example 10.
FIG. 32 shows the results of the killing of NHP iPSC-WT cells, NHP iPSC-DKO cells, and CD43 protein-overexpressing NHP iPSC-DKO+CD43 cell strain by monkey NK cells (A), along with the summary of multiple repeated experiments normalized to DKO killing results in each experiment (B), as detected by RTCA in Example 10.
FIG. 33 shows the results of the killing of NHP iPSC-WT cells, iPSC-DKO cells, and CD43 protein-overexpressing NHP iPSC-DKO+CD43 cell strain by monkey PBMCs (A), along with the summary of multiple repeated experiments normalized to DKO killing results in each experiment (B), as detected by RTCA in Example 10.
FIG. 34 shows the luminescence imaging results for transplanted cells in NHP (A) and NOG mice (B) in Example 10.
FIG. 35 shows the detection of NHP *in vivo* immune system activation, demonstrating the number of spots formed by IFN-γ secreted by monkey immune cells after injection of NHP iPSC-WT cells, NHP iPSC-DKO cells, and CD43 protein-overexpressing NHP iPSC-DKO+CD43 cells into NHP, as detected by Elispot in Example 10.

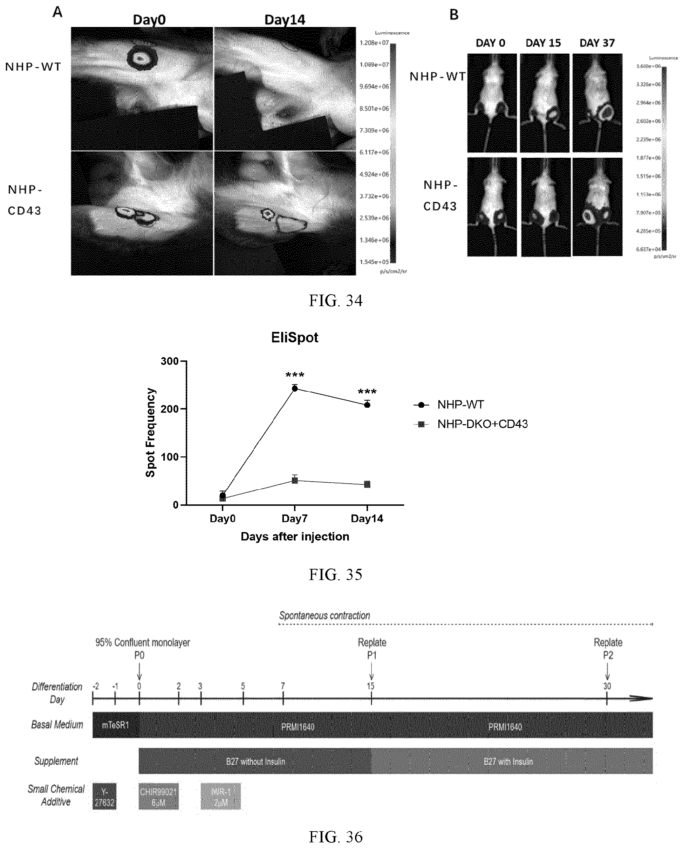

FIG. 36 shows a schematic flow chart for differentiation, culture, and compound treatment of hiPSCs-CMs in Example 11, which includes: the temporal intervals for pluripotent stem cell culture, cardiomyocyte differentiation, culture, purification, and compound treatment; the media, additives and compounds used; and the corresponding operations. Specifically, the hiPSC culture phase is from day -2 to day 0, and the medium used is mTESR1; the cardiomyocyte induction phase is from day 0 to day 15, the medium used is PRMI1640 supplemented with insulin-free B27, and the GSK3 inhibitor CHIR99021 and the Wnt inhibitor IWR-1 are employed for cell induction separately; and the cardiomyocyte maintenance phase is from day 15 to day 30, and the medium used is PRMI1640 supplemented with B27.
FIG. 37: A shows schematic bright-field micrographs and flow cytometry results for iCMs on D15 of differentiation in Example 11, and B shows schematic bright-field micrographs and flow cytometry results for iCMs on D30 of differentiation in Example 1, where the left panels display schematic bright-field micrographs, and the right panels display the cTnt-positive rates as detected by flow cytometry.
FIG. 38 shows the comparison of cardiomyocyte-related gene expression in iCMs via fluorescent quantitative PCR on day 30 of differentiation in Example 11.
FIG. 39 shows schematic diagrams of iCM calcium transient oscillations in Example 11.
FIG. 40 shows the results of cardiac function detection via echocardiography in mice with myocardial infarction in Example 11.
FIG. 41 shows the expression of HLA-I/II in various iCMs after INF-gamma stimulation, as detected by using a flow cytometer in Example 12.
FIG. 42 shows the results of the killing of WT- and DKO+CD43-differentiated iCMs by NK cells, as detected by RTCA in Example 12.
FIG. 43 shows photos demonstrating the killing of WT- and DKO+CD43-differentiated iCMs by PBMCs in Example 12.
FIG. 44 shows CFSE signal values for evaluating the PBMC-activating ability of WT- and DKO+CD43-differentiated iCMs, as detected by FACS in Example 12.

### Example 1. Construction of B2M/CIITA double knockout (DKO) cell lines

### 1. Cell culture reagents:

**Table 1**

| Reagent name | Company |
|---|---|
| mTeSR^{™} Plus | Stemcell |
| TrypLE^{™} Express Enzyme (1X), phenol red-free | ThermoFisher |
| DPBS, calcium- and magnesium-free | ThermoFisher |
| Matrigel^{®} hESC-Qualified Matrix, *LDEV-Free, 5mL | Corning^{®} |
| DMEM/F-12, GlutaMAX^{™} supplement | ThermoFisher |
| Y-27632 | Stemcell |
| TrueCut^{™} Cas9 Protein v2 | ThermoFisher |
| EasyEdit sgRNA | Genscript |

### 2. Methods and results:

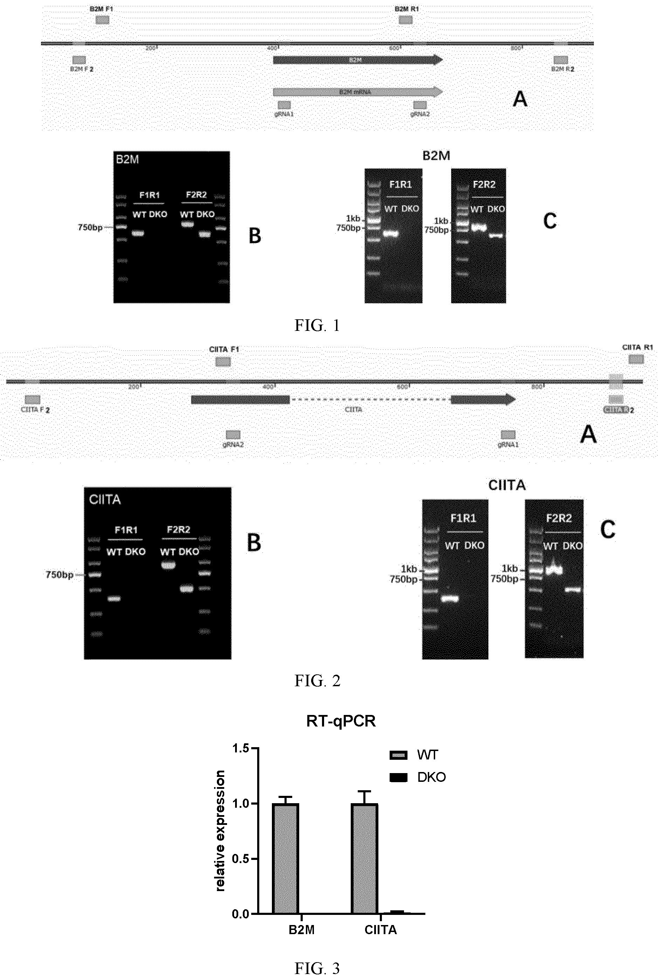

In the present disclosure, human induced pluripotent stem cells (iPSCs) were selected for the construction of target cell lines. Alternatively, the human pluripotent stem cell line H1 (Wicell, WA01) or H9 (Wicell, WA09) could also be employed for the construction. The cell culture and gene knockout reagents used are shown in Table 1. CRISPR/CAS9 was used to knock out β-2-microglobulin (B2M) in the endoplasmic reticulum, thereby preventing the formation of functional MHC-I molecules on the cell surface and thus enabling escape from killing by allogeneic CD8⁺ T cells, while escape from killing by CD4+ T cells was achieved by knocking out CIITA (a positive regulatory factor for MHC-II gene transcription) to reduce the expression of MHC class II molecules. The CRISPR/CAS9 gene knockout strategy for B2M is shown in FIG. 1. B2M-gRNA1 and B2M-gRNA2 were used to directly knock out both ends of the exons of B2M, and then genome sequence knockout validation was performed by using two pairs of PCR primers: B2M-F1/R1 and B2M-F2/R2, respectively. FIG. 1A shows the knockout strategy for the B2M gene in B2M/CIITA double knockout (DKO) cell lines, FIG. 1B shows the PCR validation results of B2M gene knockout in the H1 cell line, and FIG. 1C shows the PCR validation results of B2M gene knockout in the iPSC cell line.
gRNA sequences:
   B2M-gRNA1: CGTGAGTAAACCTGAATCTT
   B2M-gRNA2: AGTCACATGGTTCACACGGC
Identification primers
   B2M-F1: TGGGGCCAAATCATGTAGACTC
   B2M-R1: TCAGTGGGGGTGAATTCAGTGT
   B2M-F2 + B2M-R2 = 608 bp
After knockout: no band
   B2M-F2: CAGAAGTCCTTGAGAGCCTCC
   B2M-R2: TGTGCATCAGTATCTCAGCAGG
   B2M-F2 + B2M-R2 = 812 bp
After knockout: 569 bp.

In addition, the CRISPR/CAS9 gene knockout strategy for CIITA is shown in FIG. 2. CIITA-gRNA1 and CIITA-gRNA2 were used to directly knock out both ends of the exons of CIITA, and then genome sequence knockout validation was performed by using two pairs of PCR primers: CIITA-F1/R1 and CIITA-F2/R2, respectively. FIG. 2A shows the knockout strategy for the CIITA gene in B2M/CIITA double knockout (DKO) cell lines, FIG. 2B shows the PCR validation results of CIITA gene knockout in the H1 cell line, and FIG. 2C shows the PCR validation results of CIITA gene knockout in the iPSC cell line.
gRNA sequences:
   CIITA-gRNA1: GATATTGGCATAAGCCTCCC
   CIITA-gRNA2: CATCGCTGTTAAGAAGCTCC
Identification primers:
   CIITA-F1: CTGTGCCTCTACCACTTCTATG
   CIITA-R1: CCTTCCATGTCACACAACAGCC
   CIITA-F1 + CIITA-R1 = 368 bp
After knockout: no band
   CIITA-F2: TGGAATCCACACTTTCCAGTTC
   CIITA-R2: TGGAGTCTCCGTTCCTCCAG
   CIITA-F2 + CIITA-R2 = 889 bp
After knockout: 459 bp

The specific procedures were as follows:
1) Human pluripotent stem cells were routinely cultured in mTeSR1 on a Matrigel-coated 6-well plate until they reached a density of 80%. The cells were digested with TRYPLE, then neutralized with DMEM/F12, and counted. 2 × 10⁶ cells were pipetted into an EP tube and centrifuged, and then the supernatant was discarded.
2) According to the Neon transfection system's 100 µL electroporation protocol, 15 µg of TrueCut^{™} Cas9 Protein and 3 µg of gRNA (B2MgRNA1 + B2MgRNA2 + CIITA gRNA1 + CIITA gRNA2) were added to form an RNP system. The system was then uniformly mixed and left to stand at room temperature for 20 min.
3) The cells were resuspended in 100 µL of the RNP electroporation system. Electroporation was then performed using the Neon transfection system, with parameters set to 1200 V, 30 ms, and 1 pause. Immediately after electroporation, the cells were added to a pre-warmed medium and evenly seeded in one well of a Matrigel-coated 6-well plate.
4) The medium was replaced daily with a fresh mTeSR1 medium. When the single-cell colonies became visible, individual clones were picked and transferred into a 48-well plate. After clonal expansion, genome samples were collected and subjected to PCR detection to assess gene editing outcomes. The PCR results are shown in FIGs. 1 and 2. PCR-positive clones were sent to a company for Sanger sequencing for further validation.
5) The confirmed positive B2M/CIITA double allele knockout (DKO) clones were expanded, cultured, and cryopreserved.
qPCR was used to detect the expression of B2M and CIITA at the RNA level in the B2M/CIITA double allele knockout (DKO) clones. The results are shown in FIG. 3, confirming the successful knockout.
B2M-F: AAGATGAGTATGCCTGCCGT
B2M-R: ATGCGGCATCTTCAAACCTC
CIITA-F: CCTGGAGCTTCTTAACAGCGA
CIITA-R: TGTGTCGGGTTCTGAGTAGAG

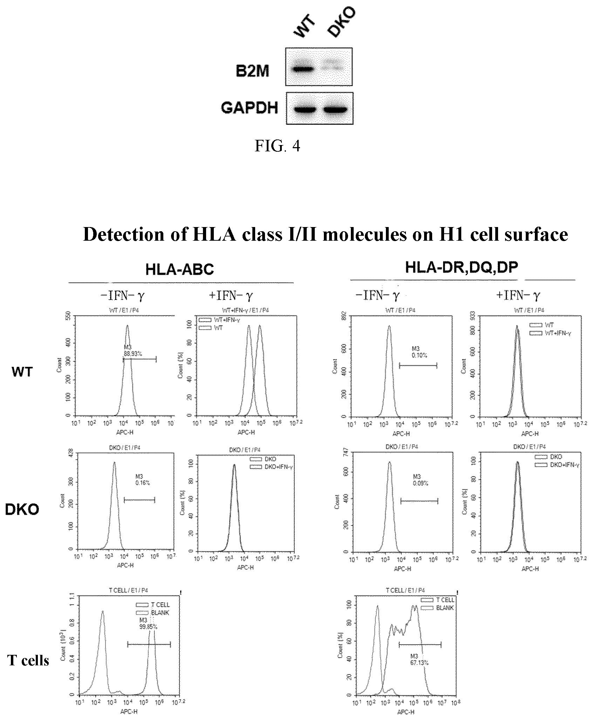

Western-Blot was used to detect the expression of B2M at the protein level in the B2M/CIITA double allele knockout (DKO) clones. The results are shown in FIG. 4, confirming the successful knockout.

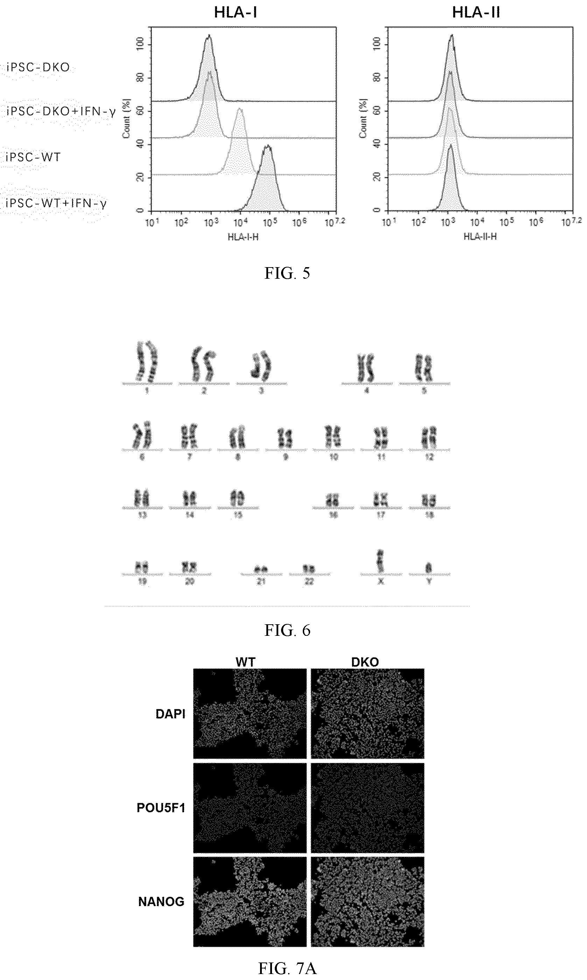

INF-gamma stimulation of WT and DKO cells: The cells were plated. During medium exchange on the following day, a medium containing INF-gamma was added to the cells. After 48 h of treatment, the cells were digested and then subjected to flow cytometry to detect HLA-I/II expression. The results are shown in FIG. 5, indicating that the DKO cells (B2M/CIITA double allele knockout stem cell positive clones) of the H1 and iPSC cell lines failed to express HLA class I/II molecules in response to INF-gamma stimulation. The upper panels show the detection results for H1 cells, the lower panels show the detection results for iPSCs. In all panels, the left side displays the detection of HLA class I molecules by HLA-ABC, and the right side displays the detection of HLA class II molecules by HLA-DR, DQ, and DP; T cells served as positive controls for the detection of HLA class I/II molecules.

Karyotype analysis of obtained B2M/CIITA double allele knockout (DKO) positive clones of H1 cells: Chromosome specimens fixed on glass slides were treated with trypsin and then stained with Giemsa stain. Metaphase chromosomes were analyzed for chromosome number and morphological structure to determine whether their karyotype was consistent with the normal karyotype. The results are shown in FIG. 6, indicating that the H1 DKO cells exhibited a normal karyotype.

### Example 2. Validation of Stemness and Immune Function of DKO Cell Lines in Example 1

### 1. Expression of pluripotency genes in WT and DKO cells

Immunofluorescence detection showed that WT and DKO cells of iPSC and H1 cell lines expressed the pluripotency genes POU5F1 and NANOG at the protein level: The cells were plated onto a 12-well plate. After the cells grew to a density of 60%-80%, the medium was aspirated, and 4% paraformaldehyde was added for fixation. After cell membrane rupture, the cells were incubated with primary antibodies against POU5F1 and NANOG at 4 °C overnight. The primary antibodies were then washed off, and fluorescence-labeled secondary antibodies were added. The mixture was incubated at room temperature, followed by photographing using a fluorescence microscope. The results are shown in FIG. 7A. RT-qPCR detection showed that H1 WT cells and H1 DKO cells expressed the pluripotency genes POU5F1, NANOG, and SOX2 at the RNA level: The results are shown in FIG. 7B (MSCs served as a negative control for pluripotency gene expression).

The flow cytometry results are shown in FIG. 8. FIG. 8A shows that both H1 WT cells and H1 DKO cells highly expressed pluripotency genes SSEA-4 and Tra1-81 on the cell surface, with percentages of 100%, 99.98% and 96.75%, 99.13%, respectively; FIG. 8B shows the flow cytometry results of the expression of pluripotency genes SSEA-4, TRA-1-60, TRA-1-81, OCT-4, and SOX2 on the surface of iPSC-WT and iPSC-DKO cells; FIG. 8C shows the immunofluorescence results of the protein levels of pluripotency genes TRA-1-60, TRA-1-81, OCT-4, SOX2, and NANOG in iPSC-WT cells; and FIG. 8D shows the immunofluorescence results of the protein levels of pluripotency genes TRA-1-60, TRA-1-81, OCT-4, SOX2, and NANOG in iPSC-DKO cells.

### 2. Differentiation ability of obtained B2M/CIITA double allele knockout (DKO) positive clones

A 100-µL suspension containing 5E+5 DKO cells was subcutaneously injected into immunodeficient mice (SCID Beige). After the teratoma volume exceeded 1.5 cm³, the teratomas were harvested, sectioned, and stained.

The obtained B2M/CIITA double allele knockout (DKO) positive clones could form teratomas *in vivo* and differentiate into cells of three germ layers (endoderm, mesoderm, and ectoderm). The results are shown in FIG. 9.

### 3. Immune function validation for DKO cells

The killing experiments of T cells and NK cells were conducted using the xCELLigence RTCA Instrument. Equivalent numbers of WT and DKO cell lines of H1 cells and iPSCs were resuspended in Essential 8 medium containing IL-2 and seeded into Matrigel-coated 96-well E-plates, and then activated T cells or NK cells were added for killing detection. RTCA detection data were analyzed using the xCELLigence software to calculate the killing rate and escape function.

**Table 2**

| **Name** | **Catalog number (Cat No.)** | **Specification** | **Manufacturer** |
|---|---|---|---|
| PE anti-human CD4 | 980804 | 500 µL/tube | Biolegend |
| APC anti-human CD8 | 980904 | 500 µL/tube | Biolegend |
| FITC anti-human CD3 | 300440 | 500 tests | Biolegend |
| APC anti-human CD16 | 301012 | 100 tests | Biolegend |
| PE anti-human CD56 (NCAM) | 318306 | 100 tests | Biolegend |
| human IL-2 | 202-1L-050/CF | 50µg | R&D |
| Y-27632 2HCl | S1049 | 5 mg | Selleck |
| Matrigel | 354277 | 5 ml | Gibco |
| Essential 8^{™} medium | A1517001 | 500 ml | Gibco |
| E-Plate VIEW 96 PET | 300601030 | 6 plates/box | Agilent |

As shown in the RTCA data in FIG. 10, the WT cells escaped killing by NK cells due to HLA-I expression, but were killed by T cells. The DKO cells could escape killing by T cells, while exhibiting heightened sensitivity to the killing by NK cells.

### Example 3. Construction and Immune Function Validation of DKO+CD47 Cell Line

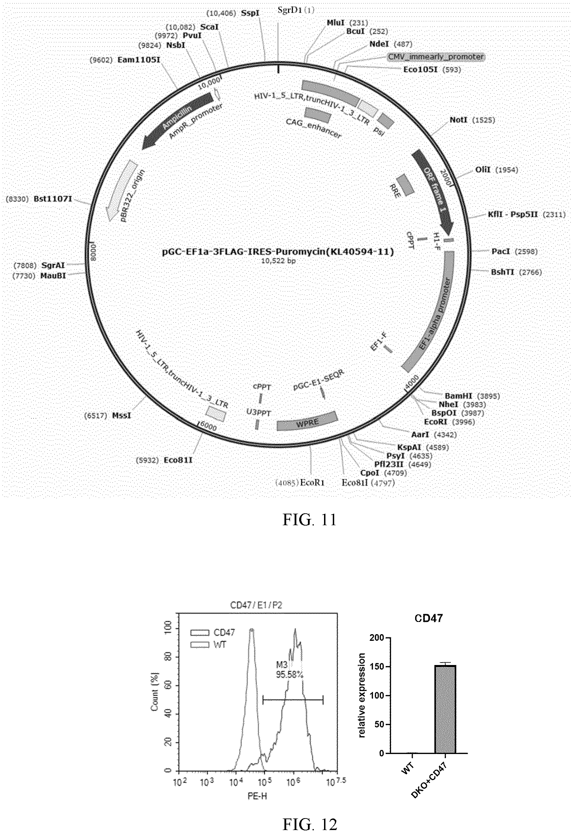

In the H1 DKO cells obtained in Example 1, CD47 (NM_198793) was overexpressed using a lentiviral vector. The amino acid sequence of CD47 is set forth in SEQ ID NO: 26. The cDNA of the overexpressed sequence (SEQ ID NO: 27) was constructed in a lentiviral plasmid (pGC-EF1a) driven by EF1a and carrying a puromycin screening marker. The structure of the pGC-EF1a plasmid is shown in FIG. 11. The plasmid was digested with *BamH*I/*Nhe*I enzymes. Upon successful ligation, Sanger sequencing was performed to validate the insertion sequence correctness, followed by viral packaging. The H1 DKO human pluripotent stem cells constructed in Example 2 were transfected. After 24 h, medium exchange was performed, and after 48 h, the medium was replaced with a puromycin-containing medium for screening. The results for the stably transfected H1 DKO+CD47 cell strain constructed above are shown in FIG. 12. After confirming correct expression, the cells were expanded and subjected to subsequent functional detection.

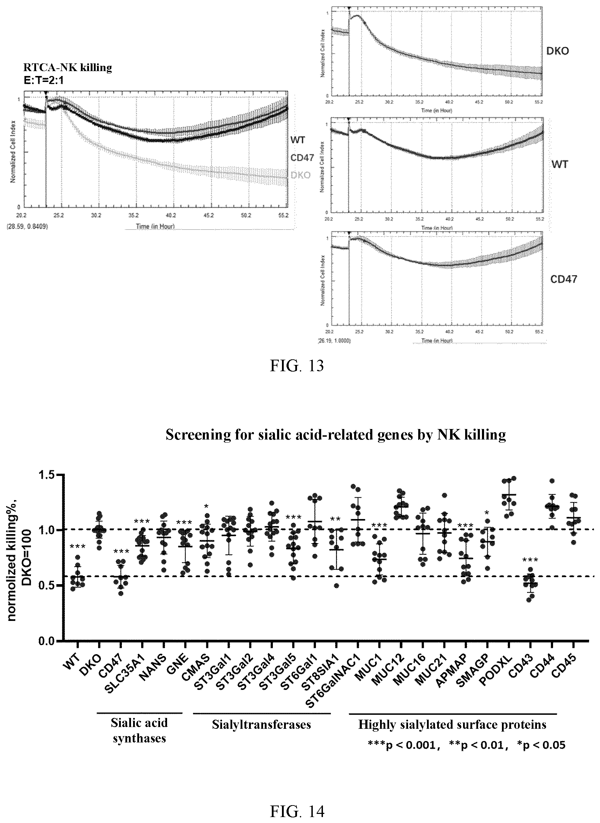

Following Example 2, RTCA detection was performed to assess whether the H1 DKO+CD47 overexpression cell strain could escape killing by NK cells successfully while escaping killing by T cells. As shown in FIG. 13, NK cells could effectively kill the H1 DKO cells, while the H1 WT cells and the H1 DKO+CD47 overexpression cells could escape killing by NK cells.

### Example 4. Screening of Universal Cells Expressing Sialic Acid-Related Molecular Proteins

Construction strategy and screening of molecular proteins in the present disclosure:
A total of 21 candidate targets were screened in this example, with their amino acid and cDNA sequences listed in Table 3. The candidate targets included sialic acid synthase genes, sialyltransferase genes, and highly sialylated cell-surface protein genes. Among these:
the sialic acid synthase genes involved were SLC35A1, NANS, GNE, and CMAS;
the sialyltransferase genes involved were ST3Gal1, ST3Gal2, ST3Gal4, ST3Gal5, ST6Gal1, ST8SIA1, and ST6GalNAC1; and
the highly sialylated cell-surface protein genes involved were MUC1, MUC12, MUC16, MUC21, APMAP, SMAGP, PODXL, CD43, CD44, and CD45.

**Table 3**

| Candidate target | Protein amino acid sequence number | cDNA nucleotide sequence Genbank number |
|---|---|---|
| SLC35A1 | SEQ ID NO.1 | NM_006416.5 |
| NANS | SEQ ID NO.2 | NM_018946.4 |
| GNE | SEQ ID NO.3 | NM_005476.7 |
| CMAS | SEQ ID NO.4 | NM_018686.6 |
| ST3Gal1 | SEQ ID NO.5 | NM_173344.3 |
| ST3Gal2 | SEQ ID NO.6 | NM_006927.4 |
| ST3Gal4 | SEQ ID NO.7 | NM_001254757.2 |
| ST3Gal5 | SEQ ID NO.8 | NM_003896.4 |
| ST6Gal1 | SEQ ID NO.9 | NM_173216.2 |
| ST8SIA1 | SEQ ID NO.10 | NM_003034.4 |
| ST6GalNAC1 | SEQ ID NO.11 | NM_018414.5 |
| MUC1 | SEQ ID NO.12 | NM_001371720.1 |
| MUC12 | SEQ ID NO.13 | BC127877.1 |
| MUC16 | SEQ ID NO.14 | AK128857.1 |
| MUC21 | SEQ ID NO.15 | NM_001010909.5 |
| APMAP | SEQ ID NO.16 | NM_020531.3 |
| SMAGP | SEQ ID NO.17 | NM_001031628.2 |
| PODXL | SEQ ID NO.18 | NM_001018111.3 |
| CD43 | SEQ ID NO.19 | NM_003123.6 |
| CD44 | SEQ ID NO.20 | NM_000610.4 |
| CD45 | SEQ ID NO.21 | NM_002838.5 |

In the H1 DKO cells obtained in Example 1, the 21 candidate targets screened in this example were separately overexpressed using lentiviral vectors. The amino acid sequences of the 21 candidate targets are shown in Table 3. The cDNAs of the overexpressed sequences (sequences as shown in Table 3) are separately constructed in lentiviral plasmids (pGC-EF1a) driven by EF1a and carrying a puromycin screening marker. The structure of the pGC-EF1a plasmid is shown in FIG. 11. The plasmids were digested with *BamH*I/*Nhe*I enzymes. Upon successful ligation, Sanger sequencing was performed to validate the insertion sequence correctness, followed by viral packaging. The H1 DKO human pluripotent stem cells constructed in Example 2 were transfected. After 24 h, medium exchange was performed, and after 48 h, the medium was replaced with a puromycin-containing medium for screening. The stably transfected cell strains of the 21 candidate targets were subjected to multiple *in-vitro* NK killing experiments (n ≥ 5), aiming to screen for novel candidate targets with optimal escape efficacy against NK cells.

The relative killing ratios in each experiment were normalized to the concurrent negative control (H1 DKO cells). A killing ratio < 1 indicates escape ability relative to DKO, with lower ratios reflecting stronger escape ability. The positive controls were WT and DKO+CD47, with the average relative killing rates across multiple killing experiments being 0.581 and 0.579, respectively, showing significant differences compared to DKO. The results demonstrated the stability of the detection platform. The screening summary results are shown in FIG. 14. Statistical analysis revealed that among the 21 genes screened above, 9 genes including SLC35A1 (0.858), GNE (0.852), CMAS (0.903), ST3Gal5 (0.834), ST8SIA1 (0.822), MUC1 (0.736), APMAP (0.744), SMAGP (0.895), and CD43 (0.520) exhibited significant differences in escape ability compared to DKO, while the remaining 12 genes showed no desirable escape ability in the *in-vitro* NK killing experiments. Among them, MUC1 (0.736), APMAP (0.744), and CD43 (0.520) exhibited superior relative killing ratios, all less than 0.75; H1 DKO+CD43 cells emerged as theoptimal cell line for escaping NK cell killing, not only showing consistently lower rates compared to DKO under multiple ratios and across repeated killing experiments, but also demonstrating a superior average killing rate compared to the positive control (H1 WT cells) and the prior art (H1 DKO+CD47 cells). Therefore, an unexpected transformation effect was achieved.

### Example 5. Construction and Stemness and Differentiation Ability Assays of DKO+CD43 Cell Line

### 1. Construction and overexpression detection of H1 DKO+CD43 cells

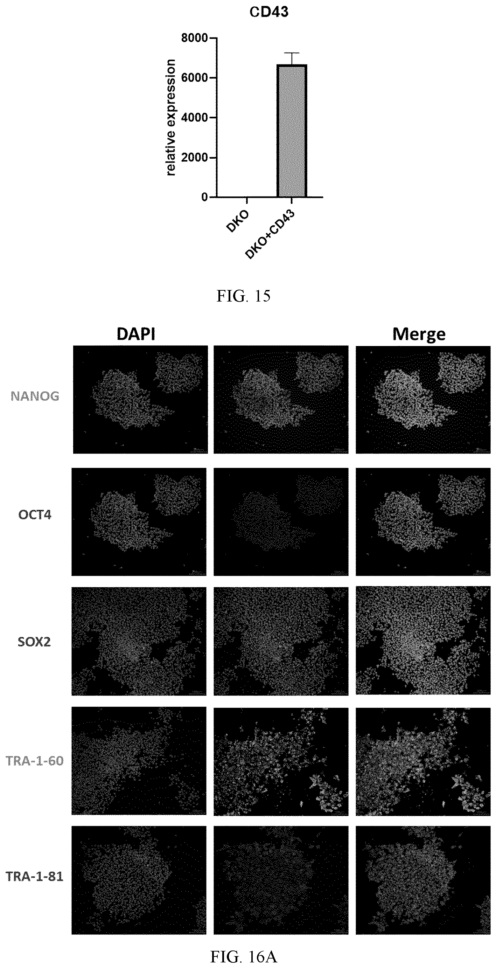

The nucleic acid sequence (NM_003123.6) encoding CD43 (the amino acid sequence of CD43 is set forth in SEQ ID NO: 19) was directly synthesized and constructed in a lentiviral plasmid (pGC-EF1a) driven by EF1a and carrying a puromycin screening marker. The structure of the pGC-EF1a plasmid is shown in FIG. 11. The plasmid was digested with *BamH*I/*Nhe*I enzymes. Upon successful ligation, Sanger sequencing was performed to validate the insertion sequence correctness, followed by viral packaging. The H1 DKO human pluripotent stem cells obtained in Example 2 were transfected, and then the medium was replaced with a puromycin-containing medium for screening. The mRNA overexpression levels in the constructed H1 DKO+CD43 cells were detected by using qPCR, with H1 DKO cells as the negative control. The results are shown in FIG. 15.
CD43 F1: GCTGGTGGTAAGCCCAGAC
CD43 R1: GGCTCGCTAGTAGAGACCAAA

### 2. Expression of pluripotency genes in H1 DKO+CD43 cells

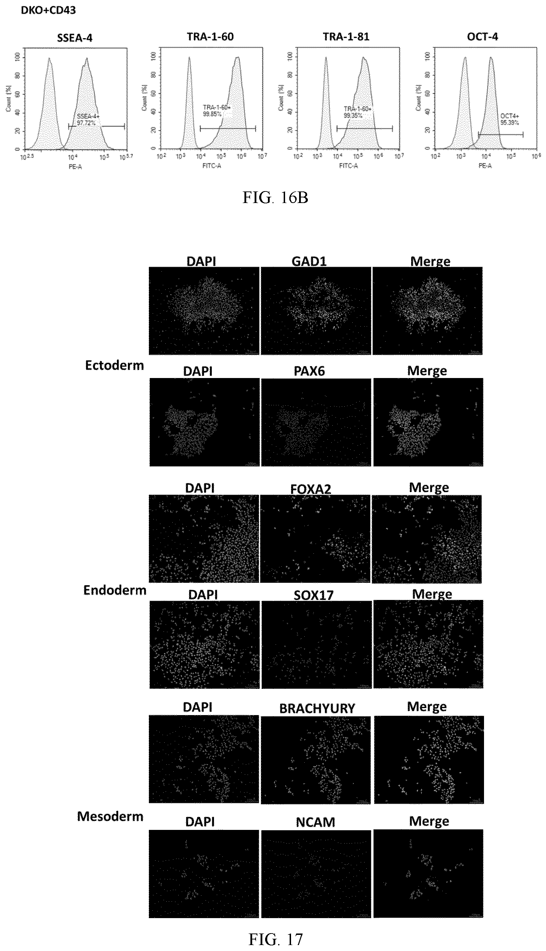

Following Example 2, immunofluorescence detection was performed. The results indicated that the H1 DKO+CD43 cells expressed the pluripotency genes NANOG, OCT4, SOX2, TRA-1-60, and TRA-1-81 at the protein level, as shown in FIG. 16A. Flow cytometry was performed, and the results indicated that the H1 DKO+CD43 cells highly expressed the pluripotency genes SSEA-4, TRA-1-60, Tra-1-81, and OCT-4 on the cell surface, with percentages of 97.72%, 99.85%, 99.35%, and 95.39%, respectively. The results are shown in FIG. 16B.

### 3. Assay for three-germ layer differentiation ability of H1 DKO+CD43 cells

DKO+CD43 cells were used for three-germ layer differentiation. To generate mesoderm, endoderm, and ectoderm cells, the dissociated H1 DKO+CD43 single cells were respectively resuspended in three germ layer media supplemented with Y27632, and an appropriate amount of cells were attached to Matrigel-coated well plates with cell climbing slices. After 24 h, the medium was replaced with a pre-warmed differentiation medium (STEMdiff^{™} Trilineage Differentiation Kit (STEMCELL, Catalog # 05230). The medium exchange was performed daily. By day 7, cells of three germ layers (mesoderm, endoderm, and ectoderm) were obtained. Immunofluorescence detection showed that the H1 DKO+CD43 cells expressed the ectoderm marker proteins PAX6 and GAD1, the mesoderm marker proteins Brachyury and NCAM, and the endoderm marker proteins SOX17 and FOXA2 at the protein level. The results are shown in FIG. 17.

### 4. Assay for teratoma formation ability of H1 DKO+CD43 cells

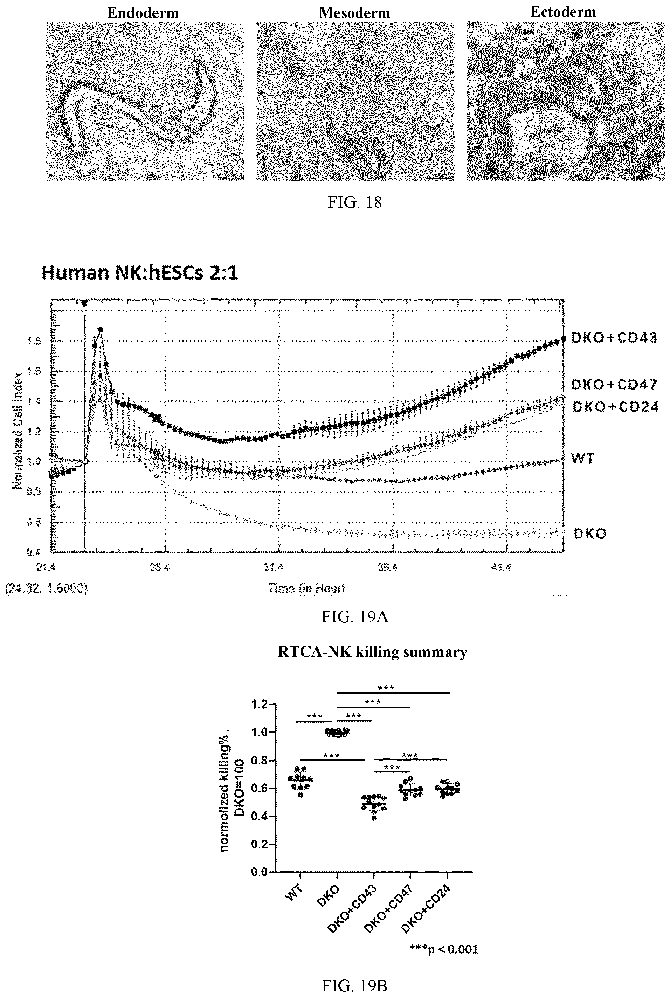

Following Example 2, a 100-µL suspension containing 5E+5 H1 DKO+43 cells was subcutaneously injected into immunodeficient mice (SCID Beige). After the teratoma volume exceeded 1.5 cm³, the teratomas were harvested, sectioned, and stained. The results are shown in FIG. 18, indicating that the H1 DKO+CD43 cells could form teratomas in mice and differentiate into cells of three germ layers (endoderm, mesoderm, and ectoderm).

### Example 6. Validation of Escape Function of H1 DKO+CD43 Cell Line Against NK Killing

This example involved the following cell lines for comparison: H1 DKO+CD47 cells and H1 DKO+CD24 cells.

The H1 DKO+CD47 cells were those used in Example 3. The H1 DKO+CD24 cells were constructed by overexpressing CD24 in DKO cells via lentiviral transfection, with the lentiviral construction and overexpression procedures referring to Example 3. The amino acid sequence of CD24 is MGRAMVARLGLGLLLLALLLPTQIYSSETTTGTSSNSSQSTSNSGLAPNPTNATTKAAGGA LQSTASLFVVSLSLLHLYS.

CD47 promotes immune escape by interacting with signal regulatory protein α (SIRPa) on the surface of immune cells. Overexpression of CD47 in B2M/CIITA double knockout (DKO) cells allows the cells to successfully escape killing by T cells and NK cells. (PMID: 32433947/PMID: 30778232).

CD24 promotes immune escape by interacting with the inhibitory receptor sialic acid-binding Ig-like lectin 10 (Siglec-10) on the surface of immune cells (PMID: 31367043). The amino acid sequence of CD24 is set forth in SEQ ID NO: 28.

### 1. Assay for escape function of H1 DKO+CD43 cells using RTCA

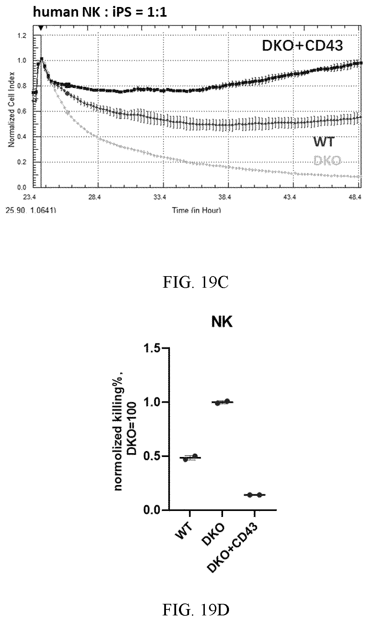

Following Example 2, NK cell killing experiments were performed using RTCA. In the experiments, the DKO cells constructed in Example 2 were completely killed by NK cells, while the H1 WT cells, H1 DKO+CD43 cells, H1 DKO+CD47 cells, and H1 DKO+CD24 cells could escape successfully. Multiple rounds of RTCA were carried out, with multiple repeated experiments normalized to DKO killing in each experiment. The summarized killing results indicated that the H1 DKO+CD43 cells exhibited the lowest killing ratio and showed significant differences compared to H1 WT cells, H1 DKO+CD24 cells, and H1 DKO+CD47 cells. The results are shown in FIGs. 19A and 19B.

### Example 7. Validation of Escape Function of DKO+CD43 Cell Line Against T+NK Mixed Lymphocyte (PBNK) Killing

### 1. Assay for the ability of H1 DKO+CD43 cells to escape PBNK using RTCA

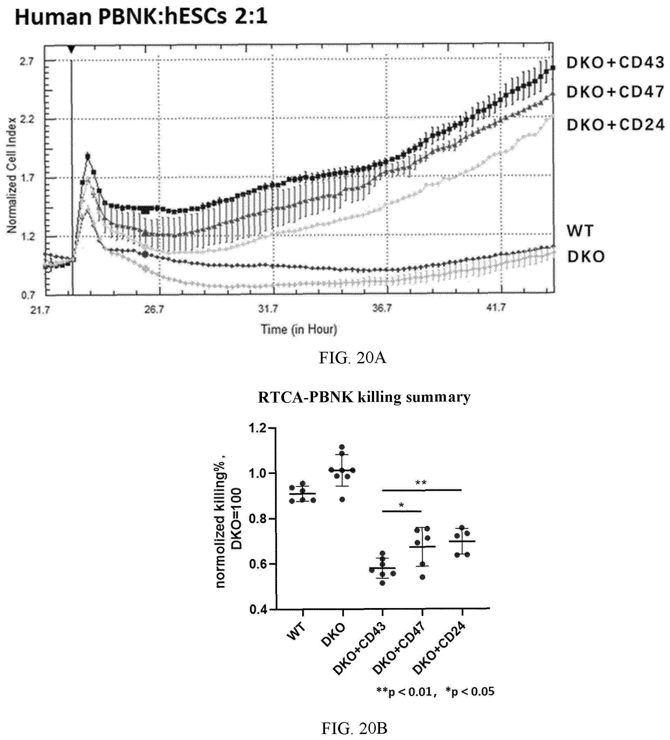

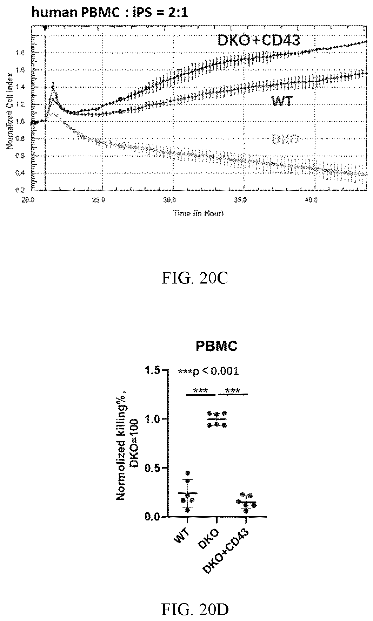

NK-activating factors were added to PBMCs in advance to improve NK cell proportion and T cell killing performance in PBMCs. The activated mixed lymphocytes (PBNK) were used as effector cells for RTCA experiments. (PMID: 33309274). Following Example 2, PBNK cell killing experiments were performed using RTCA. In the experiments, the H1 WT cells and the H1 DKO cells constructed in Example 2 were completely killed by NK cells, while the H1 DKO+CD43 cells, H1 DKO+CD47 cells, and H1 DKO+CD24 cells could escape successfully. Multiple rounds of RTCA were carried out, with multiple repeated experiments normalized to DKO killing in each experiment. The summarized killing results indicated that the H1 DKO+CD43 cells exhibited the lowest killing ratio and showed significant differences compared to the H1 DKO+CD24 cells and H1 DKO+CD47 cells. The results are shown in FIGs. 20A and 20B.

### 2. Detection of IFN-γ spot secretion in PBNK cells using Elispot

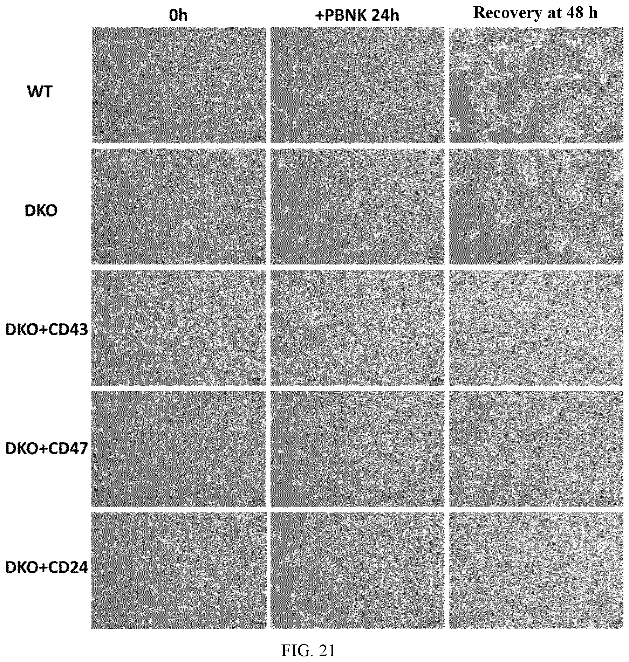

With reference to the method disclosed in "Application of Long-term cultured Interferon-γ Enzyme-linked Immunospot Assay for Assessing Effector and Memory T Cell Responses in Cattle"(PMID: 26275095), the immune escape function of DKO+CD43 cells was determined by detecting NK cell IFN-γ spot secretion via Elispot. The specific procedures were as follows:
H1 WT cells, H1 DKO cells, H1 DKO+CD43 cells, H1 DKO+CD47 cells, and H1 DKO+CD24 cells were plated onto a 96-well plate. After 24 h, the medium was discarded, and PBNK cells were added for co-culture. After 24 h, the PBNK cells were collected for subsequent IFN-γ secretion detection, and the remaining H1 WT cells, H1 DKO cells, H1 DKO+CD43 cells, H1 DKO+CD47 cells, and H1 DKO+CD24 cells after PBNK cell removal were observed. The results, as shown in FIG. 21, indicated that compared to the H1 WT cells and H1 DKO cells, fewer H1 DKO+CD43 cells, H1 DKO+CD47 cells, and H1 DKO+CD24 cells were killed by PBNK cells, indicating that the ability of the H1 DKO+CD43 cells, H1 DKO+CD47 cells, and H1 DKO+CD24 cells to escape PBNK cell killing was significantly higher than that of the WT and DKO cells.

The PBNK cells collected after 24 h were plated onto a 96-well plate coated with an IFN-γ antibody, and placed in a 37 °C incubator for incubation for 24 h. Then, an affinity antibody and streptavidin were added for incubation, followed by chromogenic detection of IFN-γ secretion spots.

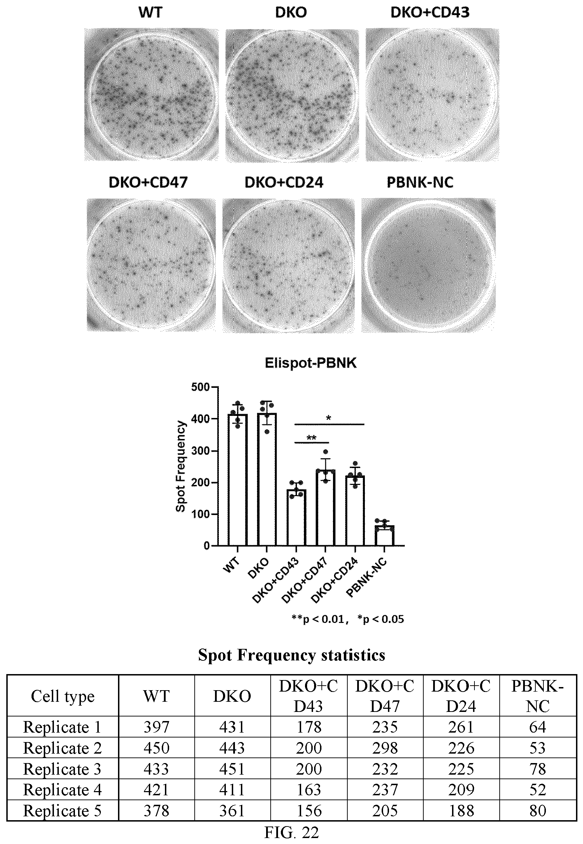

The Elispot detection results are shown in FIG. 22, where PBNK-NC represents the negative control with PBNK alone without co-culture with target cells. The number of spots formed by IFN-γ secreted by PBNK cells stimulated by co-culture with H1 DKO+CD43 cells, H1 DKO+CD47 cells, and H1 DKO+CD24 cells was similar, all being significantly lower than that of WT and DKO cells. Among them, H1 DKO+CD43 cells had the lowest number of spots, which was significantly lower than that of H1 DKO+CD47 cells and H1 DKO+CD24 cells. The results demonstrated that overexpression of CD43, CD47, and CD24 not only prevented T cell activation but also counteracted NK cell activation caused by B2M/CIITA knockout, with CD43 showing the optimal effect.

### 3. Assay for immune escape function of DKO+CD43 cells through detection of lymphocyte activation

The specific procedures were as follows:
FACS was used to detect CD107a degranulation levels to indicate whether CD8+ T and NK cells in mixed lymphocytes were activated by target cells. The target cells were seeded in a Matrigel-coated 48-well plate. After allowing the cells to adhere for 24 h, pre-activated PBNK cells were resuspended at an E:T ratio of 1:1 and then added to the 48-well plate. Simultaneously, 1 µL of Golgi transport inhibitor (Monensin) and 1 µL of BV-421-CD107a antibody were added to each well. The mixture was uniformly mixed, and then the cells were placed in a 37 °C incubator for further incubation.

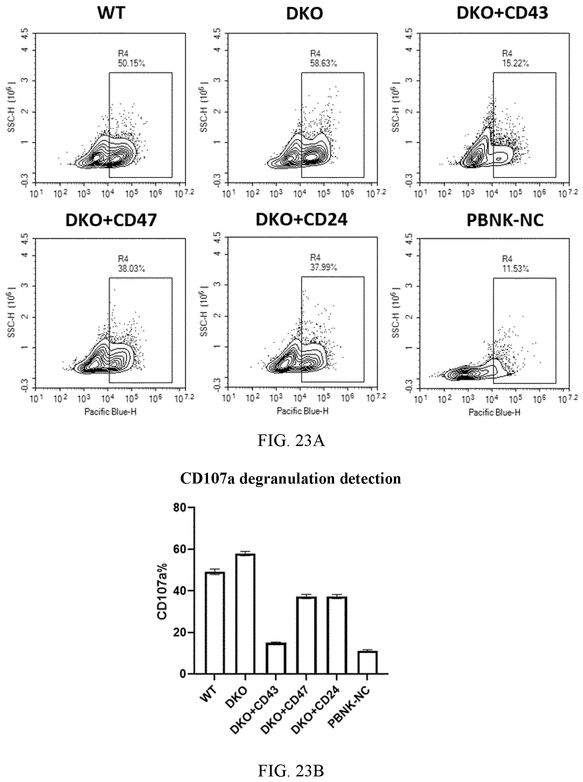

After 4 h of co-culture, the PBNK cells in the supernatant were harvested, centrifuged, then washed once with PBS, and subjected to CD107a degranulation detection. The results are shown in FIGs. 23A and 23B, indicating that compared to the DKO cells, both H1 WT cells and H1 DKO+CD43 cells were able to reduce NK cell activation.

### Example 8. Immune Escape Function of DKO+CD43 Cell Line of iPSCs

To validate the escape function of DKO+CD43 cells of iPSCs against different immune cells, experiments were performed using NK cells, PBMCs (a mixture of T cells and NK cells), and macrophages. The escape function of iPSC-DKO+CD43 cells against different immune cells was detected by RTCA. See Example 2.3 for the specific detection method.

### 1. Resistance of iPSC-DKO+CD43 cells to NK cell killing

The NK cell killing assay was performed on the XCelligence platform (ACEA BioSciences). Various types of iPSCs were separately resuspended in 100 µL of cell-specific medium and plated onto 96-well E-plates (ACEA BioSciences) coated with Matrigel (Sigma-Aldrich). After the cell index value reached 1, NK cells were added at an E:T ratio of 1:1. Data were normalized and analyzed using the RTCA software (ACEA). The results are shown in FIG. 19C, indicating that the iPSC-DKO+CD43 cells could significantly escape killing by NK cells, with superior escape efficacy compared to iPSC-WT.

### 2. Resistance of iPSC-DKO+CD43 cells to PBMC killing

NK-activating factors were added to PBMCs in advance to improve NK cell proportion and T cell killing performance in PBMCs. The activated mixed lymphocytes (PBMCs) were used as effector cells for RTCA experiments to comprehensively evaluate the immune escape ability of iPSC-DKO+CD43 cells (methodological reference PMID: 33309274). The specific procedures were as follows: The PBMC killing assay was performed on the XCelligence platform (ACEA BioSciences). Various types of iPSCs were separately resuspended in 100 µL of cell-specific medium and plated onto 96-well E-plates (ACEA BioSciences) coated with Matrigel (Sigma-Aldrich). After the cell index value reached 1, PBMC cells were added at an E:T ratio of 2:1. Data were normalized and analyzed using the RTCA software (ACEA). The results are shown in FIG. 20C, indicating that the iPSC-DKO+CD43 cells could significantly escape killing by PBMCs, with superior escape efficacy compared to iPSC-WT.

### 3. Resistance of iPSC-DKO+CD43 cells to macrophage killing

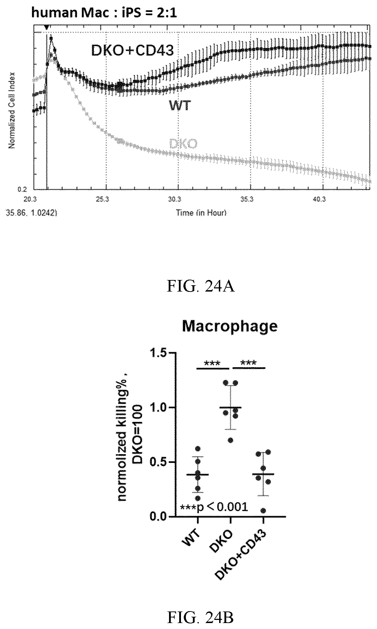

The MAC cell killing assay was performed on the XCelligence platform (ACEA BioSciences). Various types of iPSCs were separately resuspended in 100 µL of cell-specific medium and plated onto 96-well E-plates (ACEA BioSciences) coated with Matrigel (Sigma-Aldrich). After the cell index value reached 1, MAC cells were added at an E:T ratio of 2:1. Data were normalized and analyzed using the RTCA software (ACEA). The results are shown in FIG. 24, indicating that the iPSC-DKO+CD43 cells could significantly escape killing by MAC cells, with superior escape efficacy compared to the iPSC-WT and iPSC-DKO cells.

### Example 9. Validation of Immune Escape of DKO+CD43 Differentiated Cells

Cells were plated on Matrigel. When the confluence reached 40%, the medium was replaced with a differentiation medium (PMID: 22865887, Retinoic Acid-induced Differentiation of hESCs). The medium exchange was performed daily. After reaching full confluence, the cells were passaged onto a culture dish coated with 0.1% gelatin. Subculturing was performed every 3 days, during which the medium exchange was still required every day. The differentiation period was 10 days.

### 1. Assay for escape of H1 DKO+CD43 differentiated cells from NK cell killing using RTCA

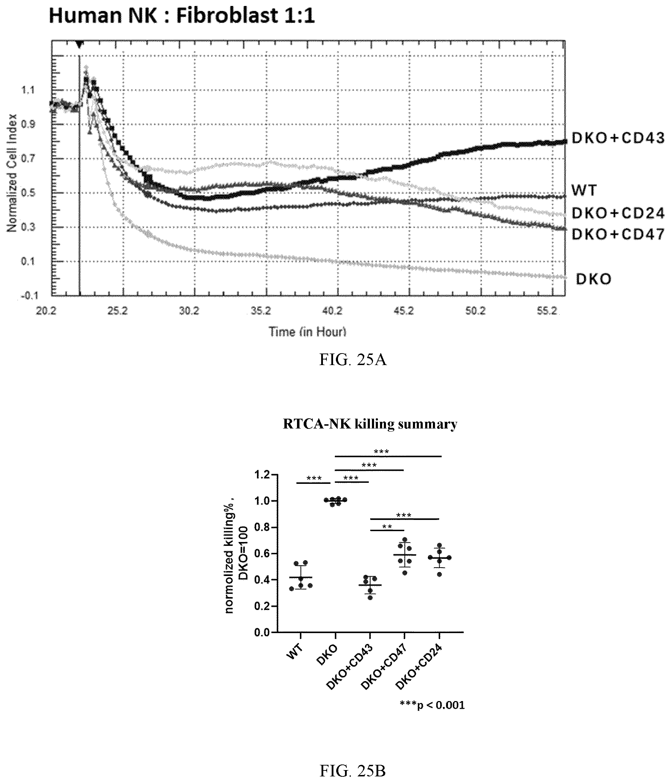

Following Example 2, NK cell killing experiments were performed using RTCA. In the experiments, the H1 WT cells and the H1 DKO+CD43, H1 DKO+CD47, and H1 DKO+CD24 differentiated cells could escape successfully. Multiple rounds of RTCA were performed, with multiple repeated experiments normalized to DKO killing in each experiment. As shown in FIGs. 25A and 25B, the summarized killing results indicated that the H1 DKO+CD43 cells exhibited the lowest killing ratio and showed significant differences compared to the H1 DKO+CD24 cells and H1 DKO+CD47 cells.

### 2. Assay for escape of H1 DKO+CD43 differentiated cells from T+NK mixed lymphocyte (PBNK) killing using RTCA

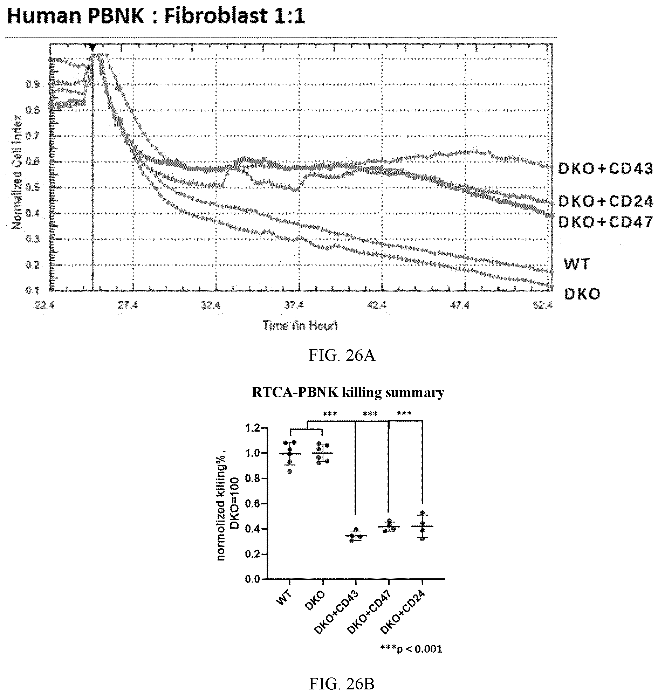

NK-activating factors were added to PBMCs in advance to improve NK cell proportion and T cell killing performance in PBMCs. The activated mixed lymphocytes (PBNK) were used as effector cells for RTCA experiments. Following Example 2, PBNK cell killing experiments were performed using RTCA. In the experiments, as shown in FIGs. 26A and 26B, the WT cells and DKO differentiated cells were completely killed by NK cells, and the H1 DKO+CD43, H1 DKO+CD47, and H1 DKO+CD24 differentiated cells could escape successfully.

### 3. Assay for escape of DKO+CD43 differentiated cells from macrophage killing using RTCA

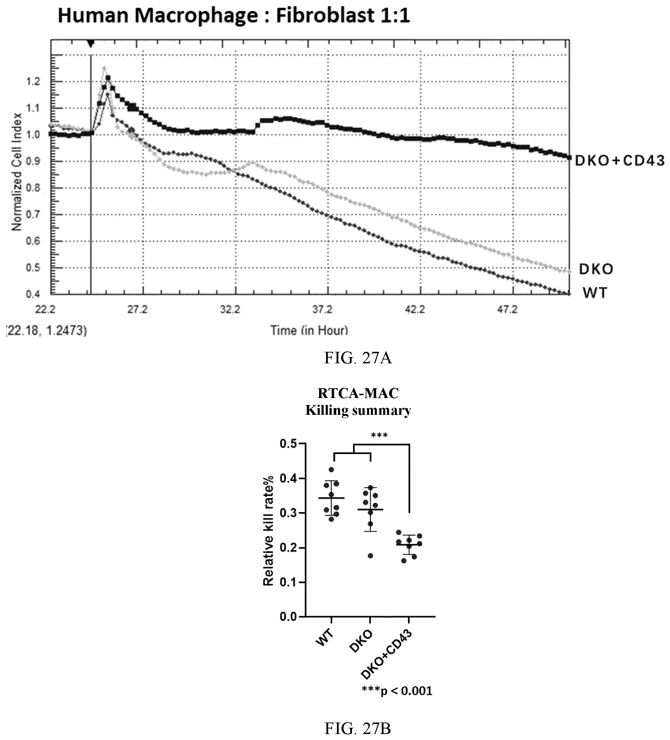

Macrophages were activated in advance to serve as effector cells for RTCA experiments. Following Example 2, macrophage killing experiments were performed using RTCA. In the experiments, as shown in FIGs. 27A and 27B, the H1 WT cells and H1 DKO differentiated cells were completely killed by macrophages (MAC), and the H1 DKO+CD43 differentiated cells could escape successfully.

### Example 10. Construction and Validation of Universal NHP Cells

This example describes a study investigating the *in-vivo* and *in-vitro* immunogenicity of universal monkey stem cells (iPSCs) in a non-human primate (NHP) model.

### 1. Construction of universal NHP cells

### a) Construction of NHP iPSC-DKO cells

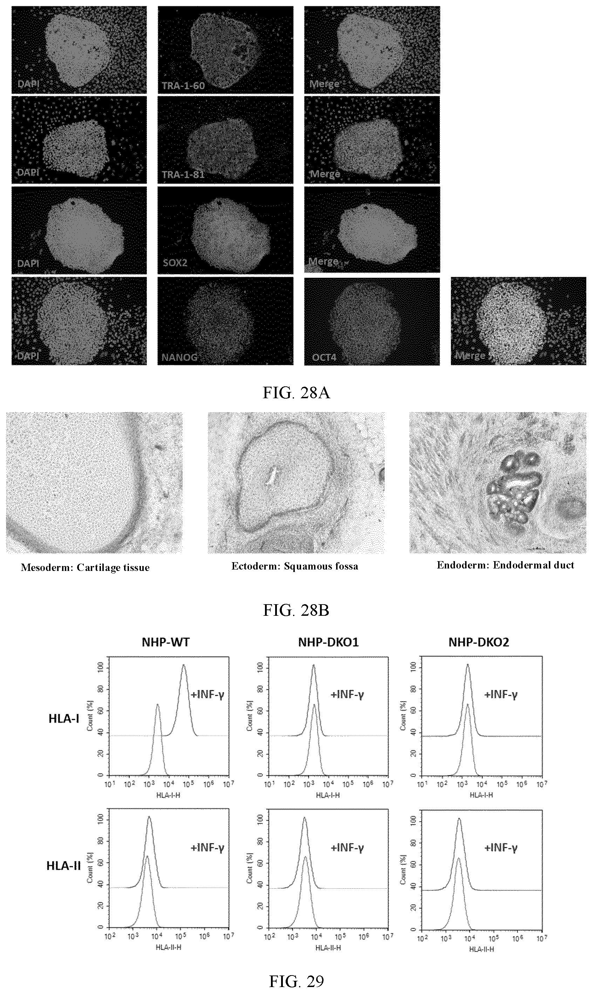

In this study, monkey iPSC-DKO cells (monkey iPSCs, prepared using the CTS^{™} CytoTune^{™}-iPS 2.1 Sendai virus reprogramming kit (Cat No.: A34546)) were generated according to the method described in Example 1. Expression of iPSC pluripotency genes and teratoma formation were detected according to the method described in Example 2, and the results are shown in FIG. 28.

NHP-B2M-gRNA1 and NHP-B2M-gRNA2 were used to directly knock out both ends of the exons of B2M, and then genome sequence knockout validation was performed by using a pair of PCR primers: NHP-B2M-F/R. NHP-CIITA-gRNA1 and NHP-CIITA-gRNA2 were used to directly knock out both ends of the exons of CIITA, and PCR validation of the genome knockout was performed through a pair of PCR primers: NHP-CIITA-F/R. Two NHP iPSC-DKO clone strains were then selected for subsequent validation work.
NHP-B2M-gRNA1 CGTGAGTAAACCTGAATCTT
NHP-B2M-gRNA2 AGTCACATGGTTCACACGGC
NHP-CIITA-gRNA1 CATCGCTGTTGAGAAGCTCC
NHP-CIITA-gRNA2 GATATTGGCATAAGCCTCCC

### B2M identification primers

NHP-B2M-FCATTTGGCCAGAGTGGAAATG
NHP-B2M-R TGGGACTCATTCAGGGTAGTA

### CIITA identification primers

NHP-CIITA-F CTGTGAGGTGACTGAGCATATC
NHP-CIITA-R GGCCAGCAATGAGCATACTA

INF-gamma stimulation of NHP iPSC-WT, NHP iPSC-DKO1, and NHP iPSC-DKO2 cells: The cells were plated onto a well plate. During medium exchange on the following day, a medium containing INF-gamma was added to the cells. After 48 h, the cells were digested and subjected to flow cytometry to detect HLA-I/II expression. NHP iPSC-DKO1 and NHP iPSC-DKO2 cells were two different clone strains generated from the same batch. The results are shown in FIG. 29, indicating that the NHP iPSC-DKO1 and NHP iPSC-DKO2 cells with B2M/CIITA double allele knockout did not express HLA-I/II and failed to up-regulate HLA-I/II in response to INF-gamma stimulation.

### b) Functional validation of escape of NHP iPSC-DKO cells from T cell killing

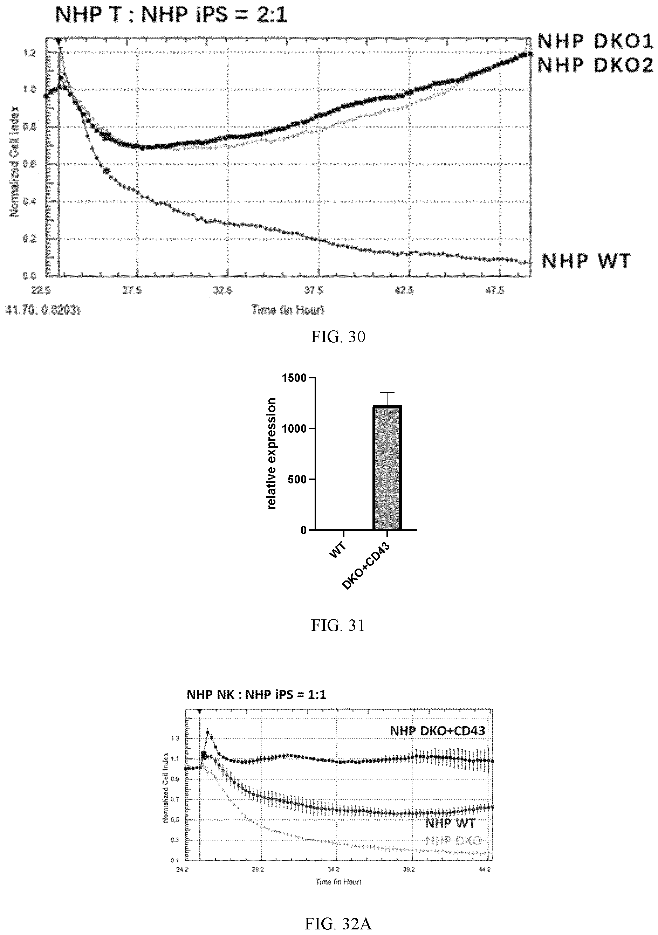

The T cell killing assay was performed on the XCelligence platform (ACEA BioSciences). NHP iPSC-WT and iPSC-DKO cells were separately resuspended in 100 µL of cell-specific medium and plated onto 96-well E-plates (ACEA BioSciences) coated with Matrigel (Sigma-Aldrich). After the cell index value reached 1, T cells were added at an E:T ratio of 2:1. Data were normalized and analyzed using the RTCA software (ACEA). The results are shown in FIG. 30, indicating that the NHP DKO cells could significantly escape killing by T cells, while the NHP iPSC-WT cells were killed.

### 2. In-vitro detection of universal NHP cells escaping immune system killing

According to the method described above, NHP iPSC-DKO+CD43 cells (monkey CD43 gene sequence: XM_005591647.3) were prepared and overexpression was confirmed by rt-qPCR. The results are shown in FIG. 31.

### a) Monkey NK killing

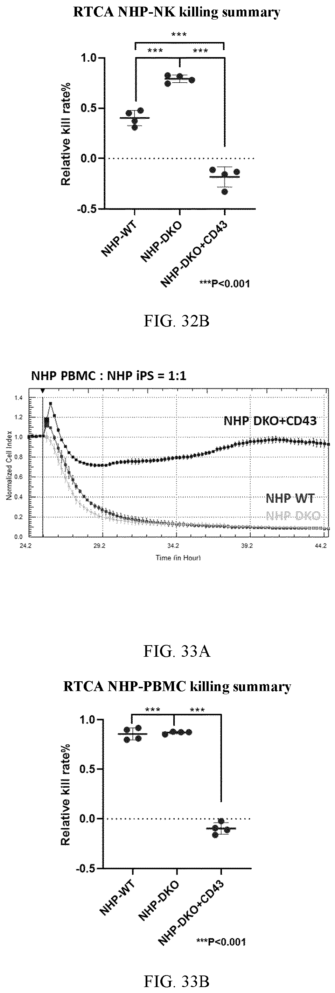

The NK cell killing assay was performed on the XCelligence platform (ACEA BioSciences). NHP iPSC-WT, NHP iPSC-DKO, and NHP iPSC-DKO+CD43 cells were separately resuspended in 100 µL of cell-specific medium and plated onto 96-well E-plates (ACEA BioSciences) coated with Matrigel (Sigma-Aldrich). After the cell index value reached 1, T cells were added at an E:T ratio of 1:1. Data were normalized and analyzed using the RTCA software (ACEA). The results are shown in FIGs. 32A and 32B, indicating that the NHP-WT and NHP iPSC-DKO+CD43 cells could significantly escape killing by monkey NK cells, and the ability to resist killing was more significant after CD43 overexpression.

### b) Monkey PBMC killing

The PBMC killing assay was performed on the XCelligence platform (ACEA BioSciences). NHP iPSC-WT, NHP iPSC-DKO, and NHP iPSC-DKO+CD43 cells were separately resuspended in 100 µL of cell-specific medium and plated onto 96-well E-plates (ACEA BioSciences) coated with Matrigel (Sigma-Aldrich). After the cell index value reached 1, PBMC cells were added at an E:T ratio of 2:1. Data were normalized and analyzed using the RTCA software (ACEA). The results are shown in FIG. 33, indicating that the NHP iPSC-DKO+CD43 cells could significantly escape killing by PBMCs, while NHP-WT and NHP-DKO were killed.

### 3. In-vivo detection of universal NHP cells escaping immune system killing

### a) Survival rate of transplanted cells

Throughout the experiment, the viability of the universal NHP cells was monitored by observing bioluminescence imaging (BLI) after cell injection in NHPs. For BLI, D-luciferin firefly potassium salt (Biosynth AG) (375 mg/kg) dissolved in sterile PBS (pH 7.4) (Gibco, Invitrogen) was injected intravenously into anesthetized monkeys (intraperitoneally into anesthetized mice). Imaging was performed on the animals using Largo (Spectral Instruments Imaging, Tucson, AZ). Bioluminescence in the region of interest (ROI) was quantified in units of maximum photons/second/square centimeter/steradian (p/s/cm²/sr).

The specific procedures were as follows: NHP iPSC-WT and NHP iPSC-DKO+CD43 cells infected with luciferase (luc)-carrying lentivirus were injected separately. The luminescent substrate D-luciferin firefly potassium salt for luc was injected intravenously. The luminescence intensity of the cells was detected using an *in-vivo* imaging system (iVIS spectrum, PerkinElmer), which could indicate the survival of the cells in the NHPs. The NHP iPSC-DKO+CD43 cells remained viable *in vivo* on day 14, while no signal was detected for NHP-WT, as shown in FIG. 34A. The same batch of cells was injected and observed via luminescence in immunodeficient NOG mice. Both NHP iPSC-WT and NHP iPSC-DKO+CD43 cells were able to grow continuously in the NOG mice, indicating that there was no difference in the *in-vivo* proliferation ability of the two cell lines, as shown in FIG. 34B. The observed difference in survival time between the NHP iPSC-DKO+CD43 cells and other cells in NHPs was due to the rejection speed of the immune system, indicating that the NHP iPSC-DKO+CD43 cells could resist immunological rejection and exhibit low immunogenicity.

### b) Degree of activation of in-vivo immune system by transplanted cells

Following the procedures in Example 7, the degree of immune activation of immune cells in the monkeys was detected by Elispot experiments on day 0, day 7, and day 14 after cell transplantation. The Elispot detection results are shown in FIG. 35, indicating the number of spots formed by IFN-γ secreted after stimulation by transplanting NHP iPSC-DKO+CD43 cells into monkeys was significantly lower than that of NHP iPSC-WT cells, suggesting that the transplanted NHP cells overexpressing CD43 almost did not activate the *in-vivo* immune system.

### Example 11. Differentiation of Universal Stem Cells into iCM Cells

### 1. Method for differentiating stem cells into iCMs in vitro

The method for differentiating iPSCs into iCMs *in vitro* is shown in FIG. 36. When hiPSCs grew to a confluence of about 95%, the cells were washed with DPBS, and then 1 mL of ethylenediaminetetraacetic acid (versene) was added. The cells were digested at 37 °C for 5 min. The versene was aspirated, and the cells were pipetted to form a single-cell suspension using 1 mL of mTeSR1 containing 10 µM Y-27632. After counting, the cells were seeded into a Matrigel-coated 12-well plate at a density of 2 × 10⁵ cells/well, which was designated as day -2 of induction. On day -1, the culture was continued for 1 day using fresh mTeSR1 without Y-27632. On day 0, when the cells grew to a confluence of about 95%, the medium was replaced with a cardiomyocyte differentiation medium for induction. The cardiomyocyte differentiation medium contained a PRMI1640 basal medium and an insulin-free B27 supplement. On days 0-2 of induction, 5 µM CHIR99021 was added to the medium. On day 2, the medium was replaced with a fresh differentiation medium. On days 3-7 of induction, 2 µM IWR-1 was added to the medium. On day 7, the medium was replaced with a fresh differentiation medium. The medium exchange was performed once every two days. On day 9 of induction, spontaneously beating cells were observed. On day 15 of differentiation, the medium was discarded, and 500 µL of trypleExpress was added to each well, followed by digestion at 37 °C for 15 min. The cells were pipetted to collect a cell suspension. The wells were then rinsed with a differentiation medium to collect residual cells. Subsequently, the cell suspension was filtered through a nylon mesh filter with a pore size of 70 µm, collected, and then centrifuged at 200 g for 5 min. The cell pellet was resuspended in a cardiomyocyte maintenance medium and then seeded into a Matrigel-coated 12-well plate at a density of 4 × 10⁵ cells/well for culture. Medium exchange was performed every two days.

### 2. Confirmation of markers for universal stem cell-derived iCMs

### a) FACS assay results

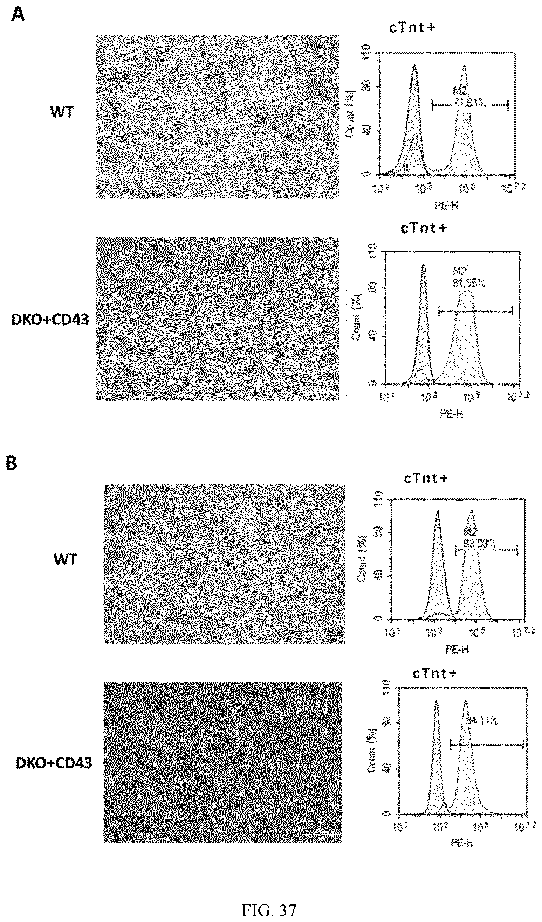

Through the above differentiation process, significant cell beating could be observed around D11. On day 15 and day 30 of differentiation, hiPSCs-CMs were digested with tryple Express for 30 min. The cell suspension was collected and centrifuged, and then the cells were washed with PBS. Subsequently, the cells were fixed with 4% PFA at room temperature for 30 min, and then blocked and permeabilized with antibodies, followed by incubation at room temperature for 10 min. Primary antibodies were diluted according to the ratio specified in the instructions and then added to the cells. The mixture was incubated at room temperature for 1 h. After washing twice with PBS to remove non-specifically bound primary antibodies, the cells were analyzed using a flow cytometer. The results are shown in FIG. 37, indicating that on D15, the proportion of cTnt+ cells in the WT group was 71.91%, while the proportion of cTnt+ cells in the iPSC DKO+CD43 cell group was 91.55%; on D30, the proportion of cTnt+ cells in the iPSC WT cell group was 93.03%, while the proportion of cTnt+ cells in the iPSC DKO+CD43 cell group was 94.11%.

### b) qPCR detection results

On day 30 of differentiation, a comparison of cardiomyocyte-related gene expression was conducted via fluorescent quantitative PCR. The procedures were as follows: The medium was aspirated. The cells were washed once with PBS, and then 500 µL of Trizol was added to each well of the 12-well plate to lyse the cells. The cell lysates were collected into RNase-free 1.5-mL EP tubes. Then, 100 µL of chloroform was added to each tube, and the mixture was shaken vigorously in a vortex oscillator for 15 s, left to stand at room temperature for 5 min, and then centrifuged at 12000 g at 4 °C for 15 min. The upper liquid was carefully pipetted into a new RNase-free 1.5-mL EP tube, and an appropriate amount of isopropanol was added. The resulting mixture was gently inverted 6 times, left to stand at room temperature for 10 min, and then centrifuged at 12000 g and 4 °C for 10 min. The supernatant was removed, and 500 µL of pre-cooled 75% ethanol was added to wash the pellet. The mixture was then centrifuged at 7500 g at 4 °C for 5 min. The ethanol was removed as much as possible, and the residue was air-dried with the tube open for 5 min. Then, 15-20 µL of DEPC water was added, and the mixture was placed on ice. After the RNA was dissolved, the RNA concentration was measured using a Nano Drop concentration measuring instrument. Reverse transcription was performed with reference to the instructions of the PrimeScript^{™} RT Master Mix kit. The reaction system was prepared on ice.

### Reaction system (20 µL):

| Reagent | Amount of use |
|---|---|
| 5×PrimeScript RT Master Mix | 4 µL |
| Total RNA | 1 µg |
| RNase Free dH₂O | Making up to 20 µL |

### Reaction program:

| Temperature | Time |
|---|---|
| 37 °C | 15 min |
| 85 °C | 5 s |
| 4 °C | hold |

The obtained cDNA could be stored at -20 °C.

The fluorescent quantitative PCR system was prepared using 2× SYBR Green qPCR Master Mix reagent.

### Reaction system (20 µL):

| Reagent | Amount of use |
|---|---|
| 2×SYBR Green qPCR Master Mix | 10 µL |
| 10 µM F+R primer | 0.4 µL |
| cDNA template | 5 µL |
| Ultra-pure water | 4.6 µL |

### Reaction program:

| | | |
|---|---|---|
| 95 °C | 5 min | |
| 95 °C | 30 s | 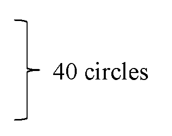 |
| 60 °C | 30 s | |
| 72 °C | 30 s | |
| 95 °C | 1 min | |
| 60 °C | 30 s | |
| 95 °C | 30 s | |

According to the Ct values provided by the software, the differences in mRNA expression folds of the samples were calculated using the △△Ct algorithm.

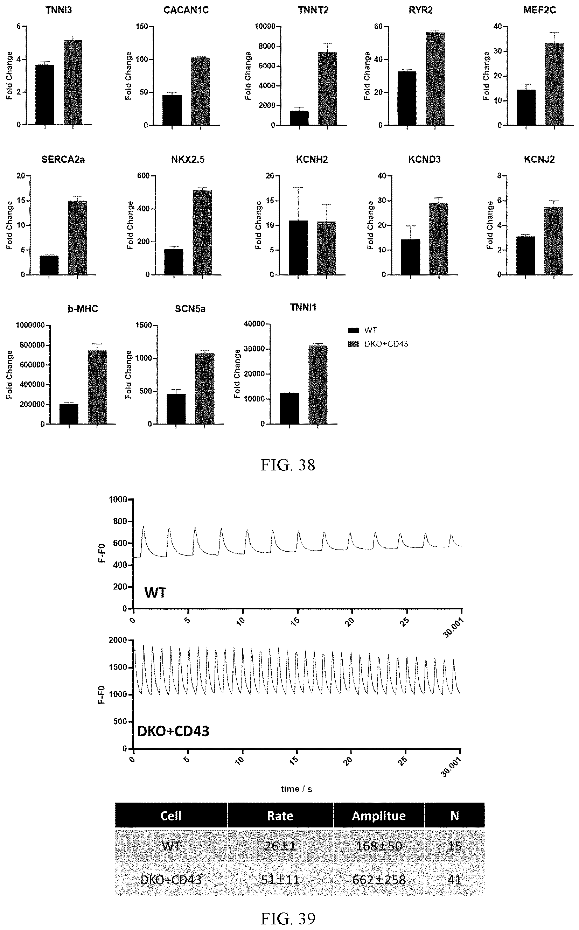

The results are shown in FIG. 38, indicating that both iPSC WT cell-differentiated iCMs and iPSC DKO+CD43 cell-differentiated iCMs showed expression of cardiomyocyte-related genes, and iPSC DKO+CD43 cell-differentiated cardiomyocytes exhibited higher relative expression levels.

### 3. Confirmation of function of universal stem cell-derived iCMs

### a) Calcium transient detection results for universal stem cell-derived iCMs

Calcium transient is one of the most representative functional parameters of cardiomyocytes. The excitation-contraction coupling in cardiomyocytes is caused by intracellular calcium ion oscillations. In adult cardiomyocytes, 70% of calcium ion influx is a result of Ca²⁺-induced Ca²⁺ release (CICR) mediated by ryanodine receptor 2 (RYR2) through the sarcoplasmic reticulum. Intracellular calcium ions are transported back to the sarcoplasmic reticulum via the sarcoplasmic/endoplasmic reticulum Ca²⁺ ATPase (SERCA), or removed from the cytoplasm through the Na⁺/Ca²⁺ exchanger (NCX). Rhod-2 AM is a fluorescent dye capable of penetrating the cell membrane. Before entering cells and being cleaved, Rhod-2 AM exhibits very weak fluorescence intensity. Upon entering cells, Rhod-2 AM is cleaved by intracellular esterases to form Rhod-2. Rhod-2 cannot penetrate the cell membrane and is thus retained within the cells. Rhod-2 then binds to calcium ions and emits fluorescence, thereby indicating dynamic changes in intracellular calcium ion concentration.

On day 30 of differentiation, the medium was discarded, and a medium containing 1 µM Rhod-2 AM was added. The cells were incubated at 37 °C for 30 min to allow Rhod-2 AM to penetrate the cell membrane and enter the cells. Excess dye was then washed away, and spontaneous calcium ion transients in the cells were recorded using the DeltaVision Ultra live cell workstation and system at 37 °C. Each recording lasted at least 30 s. As shown in FIG. 39, calcium transient recordings revealed that both iPSC WT cell-differentiated iCMs and iPSC DKO+CD43 cell-differentiated iCMs exhibited regular and ordered calcium transients, where the frequency and amplitude of calcium transients within 30 s in iPSC DKO+CD43 cell-differentiated iCMs were significantly higher than those in iPSC WT cell-differentiated iCMs.

### 4. Validation of efficacy of universal stem cell-derived iCMs in permanent ligation-induced myocardial infarction mouse model

Myocardial infarction (MI) and acute coronary heart disease are one of the most prominent causes of death among cardiovascular diseases. The MI mouse model with permanent ligation of the left anterior descending (LAD) coronary artery closely resembles human MI. The mouse surgical model of myocardial infarction induced by permanent ligation of the LAD coronary artery demonstrates high repeatability, and is a widely used method for creating mouse myocardial infarction models at present, providing a stable model foundation for our subsequent product efficacy validation. After successful MI model creation, blood flow ceases in most myocardial regions of the left ventricle. Insufficient myocardial oxygen supply leads to ischemic death of cardiomyocytes. This pathological condition triggers responses in ventricular tissue, ultimately resulting in ventricular functional defects, remodeling, and heart failure. The specific implementation steps for the mouse model with permanent ligation of the left anterior descending coronary artery were as follows:
1) The body weight of mice was measured to determine anesthetic dose and ventilator tidal volume. A heating pad was pre-warmed at 37 °C.
2) The mice were intraperitoneally injected with 1.25% tribromoethanol at a dose of 10 µL/g.
3) A depilatory cream was applied to remove hair from the throat and left thoracic area of each mouse.
4) The depth of anesthesia was checked by pinching the tail or hind paw, and the mouse was placed in a supine position on the heating pad. A small gauze dressing was placed
   under the animal's head to avoid ocular overheating. An ophthalmic gel was applied to avoid eye dryness.
5) The limbs were secured to the surface of the heating pad with adhesive tape. A 5-0 silk suture loop was passed under the upper incisors, and
   the loop ends were fixed to the heating pad with adhesive tape. The animal's mouth was kept open, and tracheal intubation was performed orally.
6) The respirator was set to a tidal volume of 2 mL and a respiratory rate of 125 breaths/min.
7) The left forepaw was released from the adhesive tape, and the mouse was carefully moved to a right position under the microscope. Once the animal was properly positioned,
   the left forelimb was secured.
8) The demarcation line between the left pectoralis minor and major muscles was identified, and an oblique incision was made along this line on the skin using scissors. Blunt dissection was performed by using
   blunt micro-scissors without incising the pectoral fascia.
9) The thoracic cavity was opened between the 3rd and 4th ribs using blunt forceps, avoiding contact with the internal thoracic artery to prevent massive hemorrhage. In addition, care was taken to avoid touching the heart or lungs.
10) The pericardium was carefully grasped and retracted using curved fine forceps (without causing damage to the heart or lungs).
11) The left anterior descending (LAD) coronary artery was identified. The LAD artery appeared as a faint reddish line extending from the left auricular edge toward the cardiac apex (or might not be visible).
12) A 7-0 silk suture was threaded beneath the LAD 2-3 mm inferior to the left atrium using a suture needle with thread. The suture was slowly tightened to avoid
   cardiac tissue tearing (this position was defined as the high ligation position, the medium ligation position was established by shifting 1-2 mm downward from the high ligation position, and the low ligation position was further established by shifting 1-2 mm downward from the medium ligation position; note: it was critical to avoid allowing the needle to enter the ventricular cavity too deeply or too shallowly). For sham-operated animals, the suture thread was passed beneath the LAD and slowly withdrawn to avoid tissue tearing. The 3rd rib was grasped with forceps, and two 5-0 silk sutures were placed under the 3rd and 4th ribs.
13) Three drops of 0.9% saline solution were placed over the opening. The expiratory exhaust pipe was closed for 2 or 3 respiratory cycles to allow normal inflation of the lungs, and then the sutures were tightened and secured.
14) The chest skin was closed with two stitches using 5-0 silk sutures, with two fixation operations.
15) After regaining consciousness, the mice were returned to their housing cages and regularly observed.
16) Four days after modeling was completed, the cells were injected at 3 points around the peri-infarct area, with an injection volume of about 5-10 µL per point,
   totaling approximately 2 million cells. The chest was then closed following the aforementioned procedures, and the mouse's condition was monitored. Two months after cell injection, cardiac functional detection was conducted via echocardiography. Echocardiography was utilized for *in-vivo* evaluation of cardiac structure and function, with measurements including left ventricular wall thickness, internal diameter, mass, fractional shortening, and ejection fraction. After the mice were subjected to depilation of the chest and abdomen and anesthetized, the following specific procedures were performed:
17) Left ventricular imaging was performed in the parasternal long-axis view using B-mode.
18) The mouse echocardiography stage was carefully adjusted to position the long axis of the left ventricle in the same plane as the ultrasound beam. During image acquisition,
   the aortic valve and left ventricle apex were positioned in the same plane as the ultrasound beam. The aortic valve and apex served as fixed points, which remained constant across serial studies in the same animal.
19) M-mode was applied on the left ventricle to perform imaging of the walls and interior of the ventricle.
20) After imaging, based on the maximum amplitude of ventricular systolic and diastolic motion, measurement points were selected to calculate the relevant characteristic values: ejection fraction (EF%), left
   ventricular internal fractional shortening (FS%), left ventricular end-systolic volume (LV Vol; s), left ventricular end-diastolic volume (LV Vol; d).

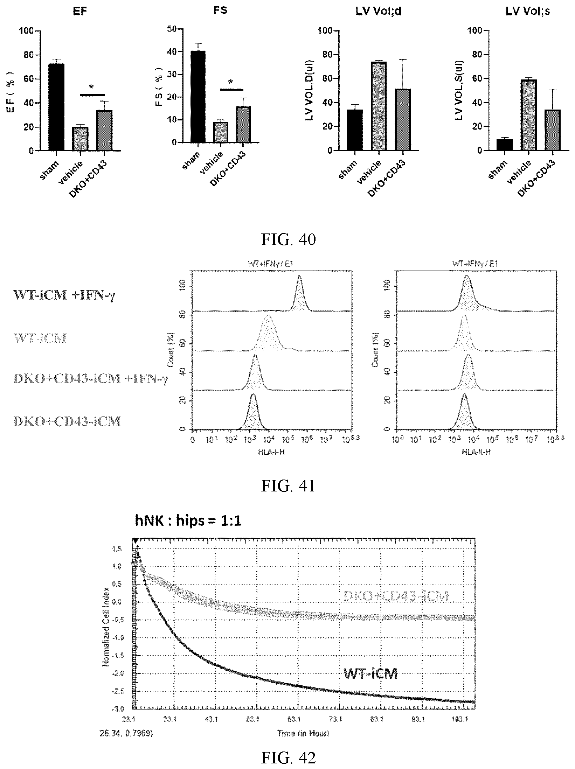

Cardiomyocytes differentiated for about 30 days were used to evaluate the efficacy in the mice with myocardial infarction. The efficacy of cardiomyocytes introduced with U66 mutation and U68 mutation in myocardial infarction treatment was compared. There were 3 mice in the sham group, 3 mice in the vehicle group, and 3 mice in the iPSC DKO+CD43 cell-differentiated iCM treatment group. The vehicle used was 5% human serum albumin and 95% compound electrolyte. As can be seen from FIG. 40, the mutated cardiomyocytes demonstrated significant efficacy in restoring cardiac function in mice, and compared with the vehicle group, the iPSC DKO+CD43 cell-differentiated iCM treatment group showed an increase in EF value from 20.34 to 34.14, an increase in FS value from 9.12 to 15.83, a decrease in end-systolic volume from 74.08 to 51.67, and a decrease in end-diastolic volume from 59.01 to 34.29.

All data in this study were presented as mean ± standard deviation (SD). Intergroup differences were calculated by Student's t-test, and a difference of P < 0.001 was considered statistically significant. All data were plotted and analyzed using the Graphpad Prism 8.0 software.

### Example 12. Validation of Low Immunogenicity of Universal Stem Cell-Derived iCMs

1. Background: For the human pluripotent stem cells selected in the present disclosure, CRISPR/CAS9 was used to knock out β-2-microglobulin (B2M) in the endoplasmic reticulum, thereby preventing the formation of functional MHC-I molecules on the cell surface and thus enabling escape from killing by allogeneic CD8+ T cells, while escape from killing by CD4+ T cells was achieved by knocking out CIITA (a positive regulatory factor for MHC-II gene transcription) to reduce the expression of MHC class II molecules. Furthermore, CD43 was expressed in B2M/CIITA double allele knockout (DKO) cells via lentivirus to escape killing by innate immunity. The successfully constructed cells, designated as iPSC DKO+CD43, served as universal stem cells. The universal stem cell-derived cardiomyocytes (iPSC DKO+CD43-iCMs) should retain the ability to avoid activating the immune system and escape its killing.
2. Validation of low immunogenicity of universal stem cell-derived iCMs

### a) Assay for low immunogenicity phenotype retention of universal cell-derived cells

INF-gamma stimulation of iCMs differentiated from iPSC WT cells and iPSC DKO+CD43 cells: The cells were plated onto a well plate. During medium exchange on the following day, a medium containing INF-gamma was added to the cells. After 48 h, the cells were digested and subjected to flow cytometry to detect HLA-I/II expression. The results are shown in FIG. 41, indicating that the iPSC DKO+CD43 stem cell positive clones with B2M/CIITA double allele knockout still did not express HLA-I/II after differentiation into iCMs, and failed to up-regulate HLA-I/II in response to INF-gamma stimulation.

### b) In-vitro detection of escape from immune system killing

### b-1. Validation of function of escape from NK cell killing

The NK cell killing assay was performed on the XCelligence platform (ACEA BioSciences, San Diego, CA). The iCMs differentiated from iPSC WT cells and iPSC DKO+CD43 cells were separately resuspended in 100 µL of cell-specific medium and plated onto 96-well E-plates (ACEA BioSciences) coated with Matrigel. After the cell index value reached 1, NK cells were added at an E:T ratio of 1:1. Data were normalized and analyzed using the RTCA software (ACEA). The results are shown in FIG. 42, indicating that only iPSC DKO+CD43-iCMs could escape killing by NK cells.

### b-2. Validation of function of escape from T+NK mixed lymphocyte (PBMC) killing

NK-activating factors were added to PBMCs in advance to improve NK cell proportion and T cell killing performance in PBMCs and maintain such improved levels. The activated mixed lymphocytes were used as effector cells for killing experiments to comprehensively evaluate the immune escape ability of iPSC DKO+CD43-iCMs.

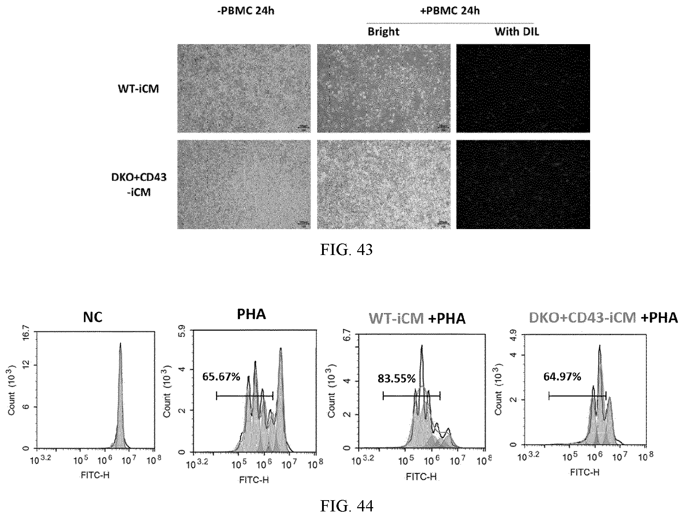

The iCMs differentiated from iPSC WT cells and iPSC DKO+CD43 cells were labeled with DIL (MCE; HY-D0083) in advance and then plated onto a 12-well plate. After 24 h, the medium was discarded, and PBMCs were added for co-culture. After 24 h, the remaining iPSC WT cell-differentiated iCMs and iPSC DKO+CD43 cell-differentiated iCMs after PBMC removal were observed. The results are shown in FIG. 43. Both bright-field and fluorescent images showed that the number of surviving cells in iPSC DKO+CD43-iCMs was significantly higher than that in iPSC WT-iCMs, indicating that the iPSC DKO+CD43-iCMs had a stronger ability to resist immune cell killing.

### c. In-vitro detection of degree of activation of immune system

The iCMs differentiated from iPSC WT cells and iPSC DKO+CD43 cells were plated onto a 12-well plate. After 24 h, the medium was discarded, and C-SFE-labeled PBMCs and PHA (PMID: 17122895) were added for co-culture, where PHA served as an activation stimulant for PBMCs. On day 5, flow cytometry was performed to determine whether iCMs stimulated PBMC proliferation. The results are shown in FIG. 44, indicating that the activated PBMC proportions in the PHA-stimulated group, iPSC WT-iCM+PHA group, and iPSC DKO+CD43-iCM group were 65.67%, 83.55%, and 64.97%, respectively. The results demonstrated that the iPSC WT-iCMs further stimulated PBMC proliferation on the basis of PHA stimulation, while the iPSC DKO+CD43-iCMs co-cultured with PBMCs failed to further stimulate PBMC proliferation.

The embodiments of the present disclosure have been described above. However, the above embodiments are not intended to limit the present disclosure. Any modification, equivalent replacement, improvement, and the like made without departing from the spirit and principle of the present disclosure shall fall within the protection scope of the present disclosure.

## Claims

1. A universal cell, relative to a wild-type cell, comprising:
1) reduced expression or absent expression of MHC-I and/or MHC-II human leukocyte antigens, and
2) an expression sequence of at least one of SLC35A1, GNE, CMAS, ST3Gal5, ST8SIA1, MUC1, APMAP, SMAGP, and CD43 genes, with the expression level of the gene being increased, wherein the cell is capable of escaping the attack by T cells and the killing by NK cells and macrophages.

2. The universal cell according to claim 1, comprising reduced expression or absent expression of MHC-I and MHC-II human leukocyte antigens.

3. The universal cell according to claim 1 or 2, further comprising a modification to increase the expression of one or more of the following polypeptides: DUX4, CD27, CD35, CD200, HLA-C, PD-L1, CD47, CD24, CD26, CCL21, Mfge8, and SerpinB9.

4. The universal cell according to claim 1 or 2, wherein a gene editing tool is used in the cell to target one or more genes encoding one or more transcription factors of MHC-I, or one or more genes encoding one or more transcription factors of MHC-II, thereby achieving the reduced expression or absent expression of MHC-I and/or MHC-II genes;
preferably, the transcription factor of MHC-I is selected from one or more of B2M, TAP1, TAP2, TAP-associated glycoprotein (Tapasin), or NLRC5; the transcription factor of MHC-II is selected from one or more of CIITA, RFXANK, RFX5, and RFXAP;
further preferably, the transcription factors are more preferably B2M and CIITA.

5. The universal cell according to claim 4, further comprising a genetic modification targeting the CIITA gene through a rare-cutting endonuclease that selectively inactivates the CIITA gene.

6. The universal cell according to any one of claims 1-5, further comprising a genetic modification targeting the B2M gene through a rare-cutting endonuclease that selectively inactivates the B2M gene.

7. The universal cell according to claim 5 or 6, wherein the rare-cutting endonuclease is selected from a CAS protein, a TALE-nuclease, a zinc finger nuclease, a meganuclease, and a homing nuclease.

8. The universal cell according to claim 7, wherein the genetic modification targeting the CIITA gene or the B2M gene through the rare-cutting endonuclease comprises a CAS protein or a polynucleotide encoding a CAS protein, and at least one guide ribonucleic acid sequence for specifically targeting the CIITA gene or the B2M gene.

9. The universal cell according to claim 8, wherein a CRISPR/CAS9 system is used to directly knock out both ends of exons of B2M and CIITA separately, wherein target sequences of the guide ribonucleic acid (gRNA) sequence for the B2M gene are SEQ ID NOs: 22 and 23, and target sequences of the guide ribonucleic acid (gRNA) sequence for the CIITA gene are SEQ ID NOs: 24 and 25.

10. The universal cell according to claim 1 or 2, wherein a gene expression-modifying molecule for one or more genes encoding one or more transcription factors of MHC-I, or a gene expression-modifying molecule for one or more genes encoding one or more transcription factors of MHC-II is introduced into the cell, thereby achieving the reduced expression or absent expression of MHC-I and/or MHC-II genes, wherein the gene expression-modifying molecule comprises one selected from an siRNA, an shRNA, a microRNA, an antisense RNA, and another RNA-mediated inhibitory molecule.

11. The universal cell according to any one of claims 1-10, wherein the amino acid sequence of the SLC35A1, GNE, CMAS, ST3Gal5, ST8SIA1, MUC1, APMAP, SMAGP, or CD43 has 70% or more homology, for example, 80% or more homology, for another example, 90% or more, 95% or more, 98% or more, or 99% or more homology, to the sequence set forth in SEQ ID NO: 1, 3, 4, 8, 10, 12, 16, 17, or 19, respectively;
further preferably, the amino acid sequence of the SLC35A1, GNE, CMAS, ST3Gal5, ST8SIA1, MUC1, APMAP, SMAGP, or CD43 is set forth in SEQ ID NO: 1, 3, 4, 8, 10, 12, 16, 17, or 19, respectively;
still further preferably, the universal cell comprises an expression sequence of at least one of MUC1, APMAP, and CD43 genes;
yet still further preferably, the universal cell comprises an expression sequence of CD43.

12. The universal cell according to any one of claims 1-11, wherein the cell is an embryonic stem cell or a pluripotent stem cell, further preferably a human stem cell or a human somatic cell.

13. A preparation method for the universal cell according to any one of claims 1-12, comprising the following steps:
1) knocking out one or more genes of one or more transcription factors of MHC-I in a stem cell; and/or
2) knocking out one or more genes of one or more transcription factors of MHC-II in the stem cell; and
3) introducing into the cell a nucleic acid sequence encoding at least one of SLC35A1, GNE, CMAS, ST3Gal5, ST8SIA1, MUC1, APMAP, SMAGP, and CD43 proteins.

14. The preparation method for the universal cell according to claim 13, wherein the transcription factor of MHC-I is selected from one or more of B2M, TAP1, TAP2, TAP-associated glycoprotein (Tapasin), or NLRC5; the transcription factor of MHC-II is selected from one or more of CIITA, RFXANK, RFX5, and RFXAP; preferably, the transcription factors are selected from B2M and CIITA.

15. The preparation method according to claim 13 or 14, wherein the knocking out in step 1) or 2) is a genetic modification targeting the CIITA gene or the B2M gene through a rare-cutting endonuclease that selectively inactivates the CIITA gene or the B2M gene;
preferably, the rare-cutting endonuclease is selected from a CAS protein, a TALE-nuclease, a zinc finger nuclease, a meganuclease, and a homing nuclease;
further preferably, the genetic modification targeting the CIITA gene or the B2M gene through the rare-cutting endonuclease comprises a CAS protein or a polynucleotide encoding a CAS protein, and at least one guide ribonucleic acid sequence for specifically targeting the CIITA gene or the B2M gene;
still further preferably, in steps 1) and 2), a CRISPR system is used to directly knock out both ends of exons of B2M and CIITA separately, wherein target sequences of gRNA for the B2M gene are SEQ ID NOs: 2 and 3, and target sequences of gRNA for the CIITA gene are SEQ ID NOs: 4 and 5.

16. The preparation method according to claim 13 or 14, wherein the knocking out in step 1) or 2) is performed by introducing a gene expression-modifying molecule for one or more genes encoding one or more transcription factors of MHC-I, or for one or more genes encoding one or more transcription factors of MHC-II, thereby achieving the reduced expression or absent expression of MHC-I and/or MHC-II genes, wherein the gene expression-modifying molecule comprises one selected from an siRNA, an shRNA, a microRNA, an antisense RNA, and another RNA-mediated inhibitory molecule.

17. The preparation method according to any one of claims 13-16, wherein in step 3), an expression vector is used to introduce into the cell the nucleic acid sequence encoding at least one of SLC35A1, GNE, CMAS, ST3Gal5, ST8SIA1, MUC1, APMAP, SMAGP, and CD43 proteins;
preferably, the expression vector used in step 3) is a viral vector;
further preferably, the viral vector is a lentivirus.

18. The preparation method according to any one of claims 13-17, wherein in step 3), the nucleic acid sequence encoding at least one of SLC35A1, GNE, CMAS, ST3Gal5, ST8SIA1, MUC1, APMAP, SMAGP, and CD43 proteins is introduced into a selected site of the stem cell; preferably, the selected site of the stem cell is a safe harbor gene site.

19. The preparation method according to any one of claims 13-18, wherein the amino acid sequence of any one of the SLC35A1, GNE, CMAS, ST3Gal5, ST8SIA1, MUC1, APMAP, SMAGP, or CD43 has 70% or more homology, for example, 80% or more homology, for another example, 90% or more, 95% or more, 98% or more, or 99% or more homology, to the sequence set forth in SEQ ID NO: 1, 3, 4, 8, 10, 12, 16, 17, or 19, respectively;
further preferably, the amino acid sequence of the SLC35A1, GNE, CMAS, ST3Gal5, ST8SIA1, MUC1, APMAP, SMAGP, or CD43 is set forth in SEQ ID NO: 1, 3, 4, 8, 10, 12, 16, 17, or 19, respectively;
still further preferably, in step 3), the expression vector is used to introduce into the cell the nucleic acid sequence encoding at least one of MUC1, APMAP, and CD43 proteins;
yet still further preferably, in step 3), the expression vector is used to introduce into the cell the nucleic acid sequence encoding CD43.

20. The preparation method according to any one of claims 13-19, wherein the universal stem cell further comprises a second expression vector comprising a polynucleotide sequence encoding one or more selected from DUX4, CD27, CD35, CD200, HLA-C, PD-L1, CD47, CD24, CD26, CCL21, Mfge8, and SerpinB9.

21. The preparation method according to claim 20, wherein the second expression vector is an inducible expression vector; preferably, the second expression vector is a viral vector.

22. A method for preparing a differentiated universal cell, comprising culturing the universal cell according to any one of claims 1-12 under differentiation conditions, thereby preparing a differentiated low-immunogenicity cell.

23. The method according to claim 22, wherein the differentiation conditions are suitable for differentiating the cell into a cell type selected from a cardiomyocyte, a nerve cell, a glial cell, an endothelial cell, a T cell, an NK cell, an NKT cell, a macrophage, a hematopoietic progenitor cell, a mesenchymal cell, an islet cell, a chondrocyte, a retinal pigment epithelial cell, a renal cell, a hepatocyte, a thyroid cell, a skin cell, a blood cell, and an epithelial cell.

24. A method of treatment, comprising administering to a patient suitable for cell therapy a population of differentiated low-immunogenicity cells prepared by the method according to claim 22 or 23.

25. A composition, comprising the universal cell according to any one of claims 1-12, preferably further comprising one or more therapeutic agents, wherein preferably, the therapeutic agent comprises a peptide, a cytokine, a checkpoint inhibitor, a mitogen, a growth factor, a small RNA, a double-stranded RNA (dsRNA), a mononuclear blood cell, a feeder cell, a feeder cell component or a replacement factor thereof, a vector comprising one or more polynucleic acids of interest, an antibody, etc.

26. A cell having increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

27. A cell having absent expression of CIITA, increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens.

28. A cell having absent expression of B2M, increased expression of any one of SLC35A1, GNE, CMAS, ST3Gal5, ST8SIA1, MUC1, APMAP, SMAGP, or CD43 protein, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

29. A cell having absent expression of CIITA and B2M, increased expression of at least one of SLC35A1, GNE, CMAS, ST3Gal5, ST8SIA1, MUC1, APMAP, SMAGP, and CD43 proteins, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

30. A cell having increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

31. A cell having absent expression of CIITA, increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

32. A cell having absent expression of B2M, increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

33. A cell having absent expression of CIITA and B2M, increased expression of at least one of SLC35A1, GNE, CMAS, ST3Gal5, ST8SIA1, MUC1, APMAP, SMAGP, and CD43 proteins, and at least one polypeptide selected from DUX4, CD27, CD35, CD200, HLA-C, PD-L1, CD47, CD24, CD26, CCL21, Mfge8, and SerpinB9, and reduced expression or absent expression of MHC class I and/or MHC class II human leukocyte antigens, wherein
preferably, the cell expresses at least one of MUC1, APMAP, and CD43 proteins;
further preferably, the cell expresses CD43.

34. The cell according to any one of claims 26-33, wherein the cell is selected from a stem cell, a differentiated cell, a pluripotent stem cell, an induced pluripotent stem cell, an adult stem cell, a progenitor cell, a somatic cell, a primary T cell, and a chimeric antigen receptor T cell.

35. Use of the universal cell according to any one of claims 1-12, the composition according to claim 25, or the cells according to claims 26-34 in the preparation of a product for cell therapy.

36. Use of the universal cell according to any one of claims 1-12, the composition according to claim 25, or the cells according to claims 26-34 in the preparation of a product for organ transplantation.

37. Use of the universal stem cell according to any one of claims 1-12, the composition according to claim 25, or the cells according to claims 26-34 in the construction of a cell bank of universal PSCs.

38. Use of the universal stem cell according to any one of claims 1-12, the composition according to claim 25, or the cells according to claims 26-34 as a gene drug carrier.

39. A kit for obtaining an isolated low-immunogenicity cardiomyocyte by *in vitro* differentiation from the universal stem cell according to any one of claims 1-12, comprising a first medium and a second medium, wherein
the first medium is a basal medium comprising a GSK-3 inhibitor;
the second medium is a basal medium comprising a Wnt inhibitor.

40. The kit according to claim 39, wherein the first medium or the second medium comprises a PRMI1640 basal medium and an insulin-free B27 supplement; and/or
the GSK-3 inhibitor is selected from CHIR99021; and/or
the Wnt inhibitor is selected from any one, two, or more of IWR-1, IWP-2, IWP-4, and C59; and/or
the concentration of the GSK-3 inhibitor is 0.5 µM-10 µM, preferably 6 µM; and/or
the concentration of the Wnt inhibitor is 0.5 µM-20 µM, preferably 2 µM.

41. A method for obtaining an isolated low-immunogenicity cardiomyocyte by *in vitro* differentiation from the universal stem cell according to any one of claims 1-12, wherein the universal stem cell has eliminated endogenous β-2 microglobulin (B2M) gene activity and endogenous class II transactivator (CIITA) gene activity, and increased expression of CD43 protein; the method further comprises the following steps:
(a) culturing the stem cells in a basal medium supplemented with a GSK-3 inhibitor to induce differentiation of the stem cells into cardiomyocytes;
(b) continuing the induced differentiation in a basal medium;
(c) continuing the induced differentiation in a basal medium supplemented with a Wnt inhibitor; and
(d) continuing the induced differentiation in a basal medium;
further preferably, the method comprises the following steps:
(a) on days 0-2, culturing the stem cells in a first medium supplemented with a GSK-3 inhibitor to induce differentiation of the stem cells into cardiomyocytes;
(b) after 1 day of induction, continuing the induced differentiation in a basal medium;
(c) on days 3-5, continuing the induced differentiation in a second medium supplemented with a Wnt inhibitor; and
(d) on days 5-15, continuing the induced differentiation in a basal medium.

42. The method according to claim 41, wherein
in steps (a), (b), (c), and (d), the first medium and the second medium comprise a PRMI1640 basal medium and an insulin-free B27 supplement; and/or
the GSK-3 inhibitor is selected from CHIR99021; and/or
the Wnt inhibitor is selected from any one, two, or more of IWR-1, IWP-2, IWP-4, and C59; and/or
the concentration of the GSK-3 inhibitor is 0.5 µM-10 µM, preferably 6 µM; and/or
the concentration of the Wnt inhibitor is 0.5 µM-20 µM, preferably 2 µM; and/or
in step (a), when the universal stem cells grow to a confluence of 85%-98% (e.g., 95%), the medium is replaced with the first medium to induce differentiation.

43. The method according to claim 41, wherein the basal medium is selected from a PRMI1640 medium, an mTESR1 medium, etc.; further preferably, the basal medium is a PRMI1640 medium supplemented with insulin-containing B27.

44. A cell therapeutic agent for preventing or treating a heart disease, comprising the differentiated cardiomyocyte prepared by the method according to any one of claims 41-43.

45. The cell therapeutic agent according to claim 44, wherein the heart disease may be at least one selected from the group consisting of, but not limited to: myocardial infarction, angina pectoris, ischemic cardiomyopathy, primary cardiomyopathy, secondary cardiomyopathy, and heart failure.
